# EUROPÄISCHE PATENTANMELDUNG

(11) **EP 0 671 391 A1**
(43) Veröffentlichungstag der Anmeldung: **13.09.1995**
(21) Anmeldenummer: 95102762.2
(22) Anmeldetag: 27.02.1995
(51) Int. Cl.: C07D 215/56, C07D 401/04, C07D 471/04, C07D 487/04, C07D 487/14, C07D 487/08, C07D 491/04, A61K 31/47

(54) **5-Vinyl- und 5-Ethinyl-chinolon- und naphthyridon-Carbonsäuren**

(30) Priorität: 11.03.1994 DE 4408212
(71) Anmelder: BAYER AG, 51368 Leverkusen (DE)
(72) Erfinder: Himmler, Thomas, Dr., D-51519 Odenthal (DE); Petersen, Uwe, Dr., D-51375 Leverkusen (DE); Bremm, Klaus-Dieter, Dr., D-45661 Recklinghausen (DE); Endermann, Rainer, Dr., D-42113 Wuppertal (DE); Stegemann, Michael, Dr., D-51381 Leverkusen (DE); Wetzstein, Heinz-Georg, Dr., D-51377 Leverkusen (DE)

(57) **Zusammenfassung**

Die Erfindung betrifft neue 5-Vinyl- und 5-Ethinyl-chinolon- und -naphthyridon-carbonsäuren, Verfahren zu ihrer Herstellung sowie diese enthaltende antibakterielle Mittel und Futterzusatzstoffe.

## Beschreibung

Die Erfindung betrifft neue 5-Vinyl- und 5-Ethinyl-chinolon- und naphthyridoncarbonsäuren, Verfahren zu ihrer Herstellung sowie diese enthaltende antibakterielle Mittel und Futterzusatzstoffe.

Es ist bereits bekannt geworden, daß 5-Alkyl-chinoloncarbonsäuren antibakteriell wirksam sind: So wurden 5-Alkyl-chinoloncarbonsäuren zum Beispiel in der DE 3 910 663, EP 319 906, EP 287 951 und WO 8 906 849 beschrieben.

Es wurde nun gefunden, daß die neuen Verbindungen der Formel (I) 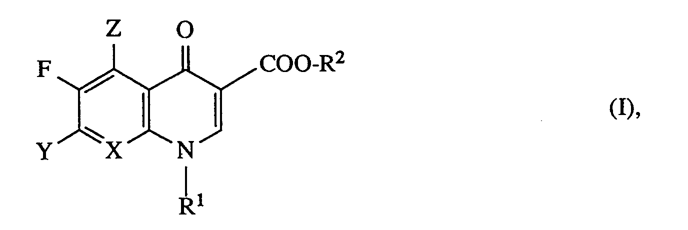
in welcher
- R¹: für gegebenenfalls durch Hydroxy, Halogen oder C₁-C₃-Alkoxy substituiertes geradkettiges oder verzweigtes C₁-C₄-Alkyl, für gegebenenfalls durch Halogen oder C₁-C₃-Alkyl substituiertes C₃-C₆-Cycloalkyl, C₂-C₄-Alkenyl, ferner für C₁-C₃-Alkoxy, Amino, Monoalkylamino mit 1 bis 3 C-Atomen, Dialkylamino mit 2 bis 6 C-Atomen oder für gegebenenfalls durch Halogen ein- bis dreifach substituiertes Phenyl steht,
- R²: für Wasserstoff, Alkyl mit 1 bis 4 Kohlenstoffatomen oder (5-Methyl-2-oxo-1,3-dioxol-4-yl)-methyl steht,
- X: für eine Gruppierung CH, C-Halogen, COCH₃, COCHF₂, C-CH₃ oder N steht,
- Z: für 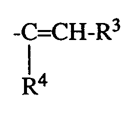 oder -C≡C-R⁵ steht,
worin
- R³: Wasserstoff, C₁-C₃-Alkyl, C₁-C₃-Alkoxy oder Alkoxymethyl mit 1 bis 3 C-Atomen im Alkoxyteil,
- R⁴: Wasserstoff oder Halogen und
- R⁵: Wasserstoff, gegebenenfalls durch Halogen oder Hydroxy ein- bis dreifach substituiertes C₁-C₆-Alkyl, C₂-C₃-Alkenyl, Alkoxy mit 1 bis 3 C-Atomen, Alkoxymethyl mit 1 bis 3 C-Atomen im Alkoxyteil, Halogen oder Trimethylsilyl bedeutet und
- Y: für 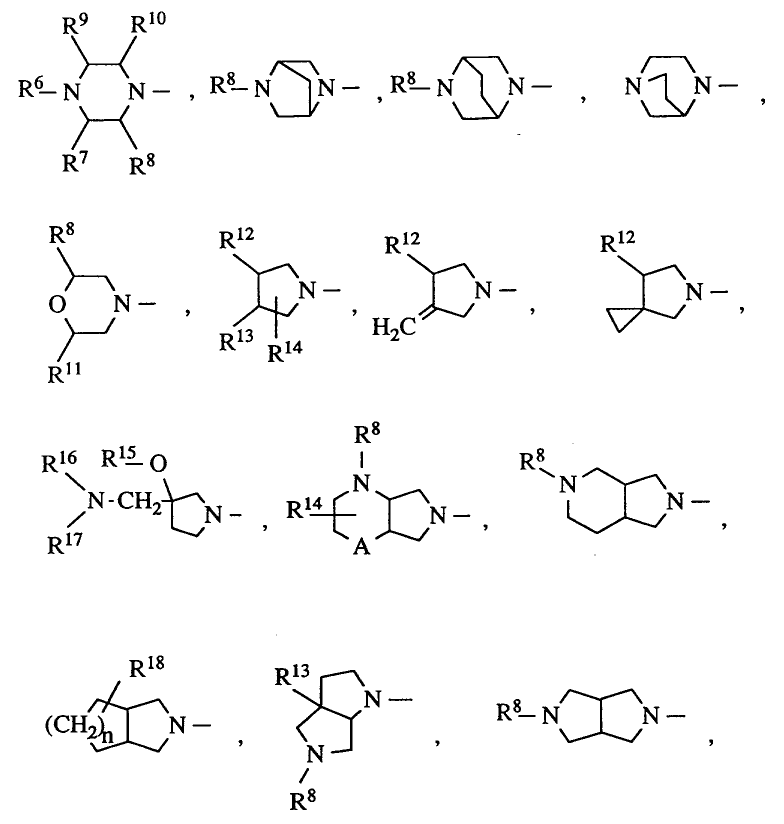 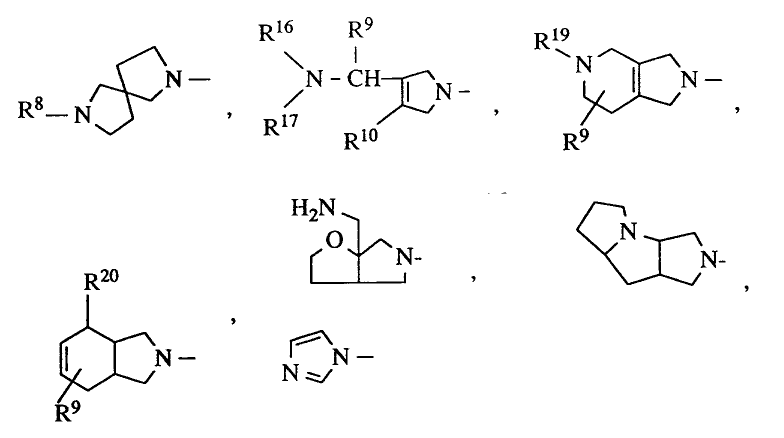 oder 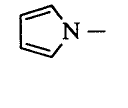 steht,
worin
- R⁶: für Wasserstoff, gegebenenfalls durch Hydroxy oder Methoxy substituiertes geradkettiges oder verzweigtes C₁-C₄-Alkyl, Cyclopropyl, Oxoalkyl mit 1 bis 4 C-Atomen, Acyl mit 1 bis 3 C-Atomen,
- R⁷: für Wasserstoff, Methyl, Phenyl, Thienyl oder Pyridyl,
- R⁸: für Wasserstoff oder Methyl,
- R⁹: für Wasserstoff oder Methyl,
- R¹⁰: für Wasserstoff oder Methyl,
- R¹¹: für Wasserstoff, Methyl oder 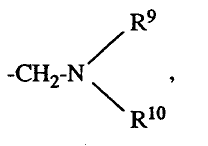
- R¹²: für Wasserstoff, Methyl, Amino, gegebenenfalls durch Hydroxy substituiertes Alkyl oder Dialkylamino mit 1 oder 2 C-Atomen im Alkylteil, Aminomethyl, Aminoethyl, gegebenenfalls durch Hydroxy substituiertes Alkyl- oder Dialkylaminomethyl mit 1 oder 2 C-Atomen im Alkylteil oder 1-Imidazolyl,
- R¹³: für Wasserstoff, Hydroxy, Methoxy, Methylthio oder Halogen, Methyl, Hydroxymethyl,
- R¹⁴: für Wasserstoff oder Methyl,
- R¹⁵: für Wasserstoff, Methyl oder Ethyl,
- R¹⁶: für Wasserstoff, Methyl oder Ethyl,
- R¹⁷: für Wasserstoff, Methyl oder Ethyl,
- R¹⁸: für Hydroxy, 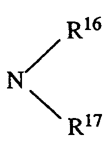 oder 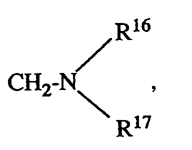
- R¹⁹: für Wasserstoff, gegebenenfalls durch Hydroxy substituiertes C₁-C₃-Alkyl, Alkoxycarbonyl mit 1 bis 4 Kohlenstoffatomen im Alkoxyteil oder C₁-C₃-Acyl,
- R²⁰: für Wasserstoff, Hydroxy, 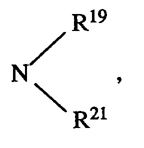 Hydroxymethyl oder 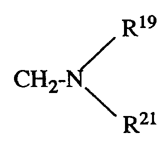 steht, worin
- R²¹: Wasserstoff oder Methyl bedeutet,
- A: für CH₂, O oder für eine direkte Bindung steht und
- n: für 1 oder 2 steht,
und deren pharmazeutisch verwendbaren Hydrate und Säureadditionssalze sowie die Alkali-, Erdalkali-, Silber- und Guanidiniumsalze der zugrundeliegenden Carbonsäuren eine hohe antibakterielle Wirkung aufweisen.

Sie eignen sich daher als Wirkstoffe für die Human- und Veterinärmedizin, wobei zur Veterinärmedizin auch die Behandlung von Fischen zur Therapie oder Vorbeugung bakterieller Infektionen zu zählen ist.

Bevorzugt sind die Verbindungen der Formel (I),
in welcher
- R¹: für gegebenenfalls durch Hydroxy oder Halogen substituiertes C₁-C₂-Alkyl, C₃-C₅-Cycloalkyl, Vinyl, Amino, Monoalkylamino mit 1 bis 2 C-Atomen, Dialkylamino mit 2 bis 4 C-Atomen oder für gegebenenfalls durch Halogen ein- oder zweifach substituiertes Phenyl steht,
- R²: für Wasserstoff, Alkyl mit 1 bis 3 Kohlenstoffatomen oder (5-Methyl-2-oxo-1,3-dioxol-4-yl)-methyl steht
- X: für eine Gruppierung CH, CF, CCl, COCH₃ oder N steht,
- Z: für 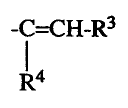 oder -C≡C-R⁵ steht,
worin
- R³: Wasserstoff, C₁-C₂-Alkyl, Methoxy oder Methoxymethyl,
- R⁴: Wasserstoff und
- R⁵: Wasserstoff, gegebenenfalls durch Fluor ein- bis dreifach substituiertes C₁-C₄-Alkyl, C₂-C₃-Alkenyl, Methoxy oder Trimethylsilyl bedeutet und
- Y: für 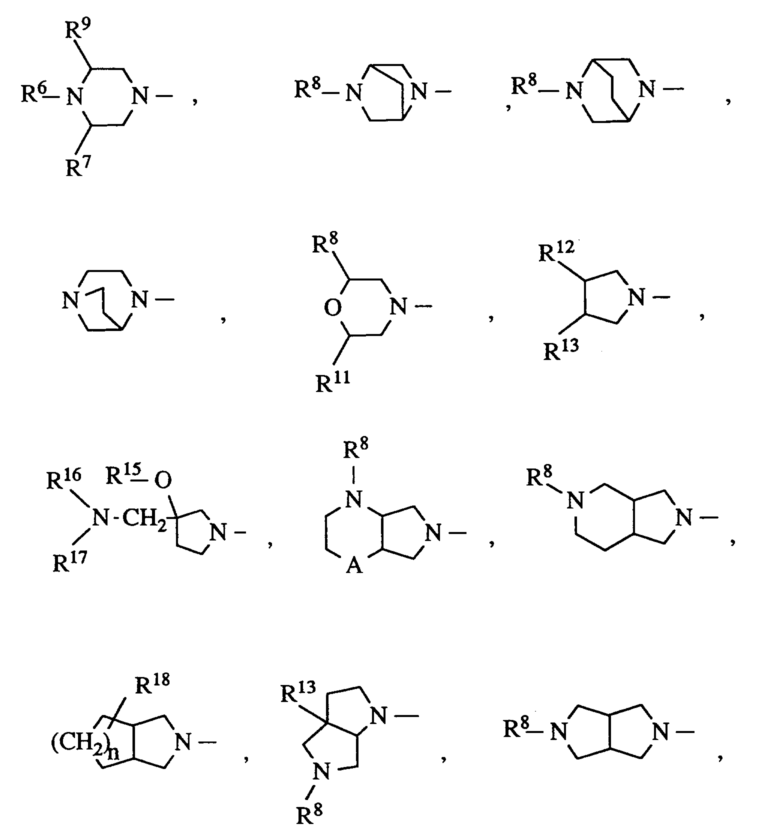 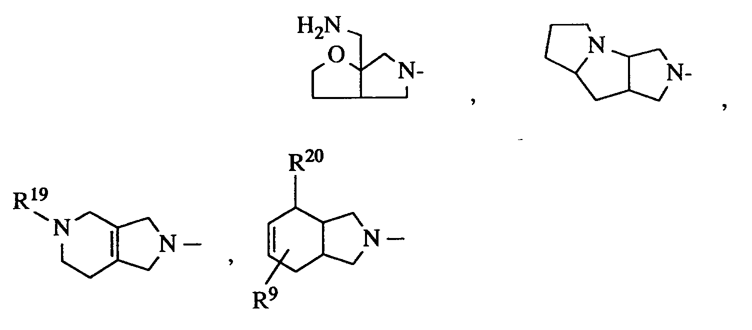 oder 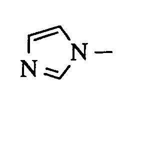 steht,
worin
- R⁶: für Wasserstoff, gegebenenfalls durch Hydroxy substituiertes geradkettiges oder verzweigtes C₁-C₃-Alkyl, Oxoalkyl mit 1 bis 4 C-Atomen,
- R⁷: für Wasserstoff, Methyl oder Phenyl,
- R⁸: für Wasserstoff oder Methyl,
- R⁹: für Wasserstoff oder Methyl,
- R¹¹: für Wasserstoff, Methyl oder -CH₂-NH₂,
- R¹²: für Wasserstoff, Methyl, Amino, Methylamino, Dimethylamino, Aminomethyl, Methylaminomethyl oder Ethylaminomethyl,
- R¹³: für Wasserstoff, Hydroxy, Methoxy, Fluor, Methyl oder Hydroxymethyl,
- R¹⁵: für Wasserstoff oder Methyl,
- R¹⁶: für Wasserstoff oder Methyl,
- R¹⁷: für Wasserstoff oder Methyl,
- R¹⁸: für 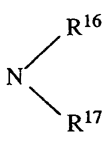 oder 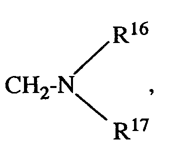
- R¹⁹: für Wasserstoff, Methyl oder Ethyl,
- R²⁰: für 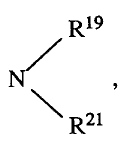 steht,
worin
- R²¹: Wasserstoff oder Methyl bedeutet,
- A: für CH₂, O oder für eine direkte Bindung steht und
- n: für 1 oder 2 steht.

Besonders bevorzugt sind Verbindungen der Formel (I),
in welcher
- R¹: für Methyl, Ethyl, Cyclopropyl, Fluorcyclopropyl oder gegebenenfalls durch Fluor ein- oder zweifach substituiertes Phenyl,
- R²: für Wasserstoff, Methyl oder Ethyl,
- X: für eine Gruppierung CH, CF oder CCl steht,
- Z: für -CH=CH₂ oder -C≡C-R⁵ steht,
worin
- R⁵: Wasserstoff oder Trimethylsilyl bedeutet und
- Y: für 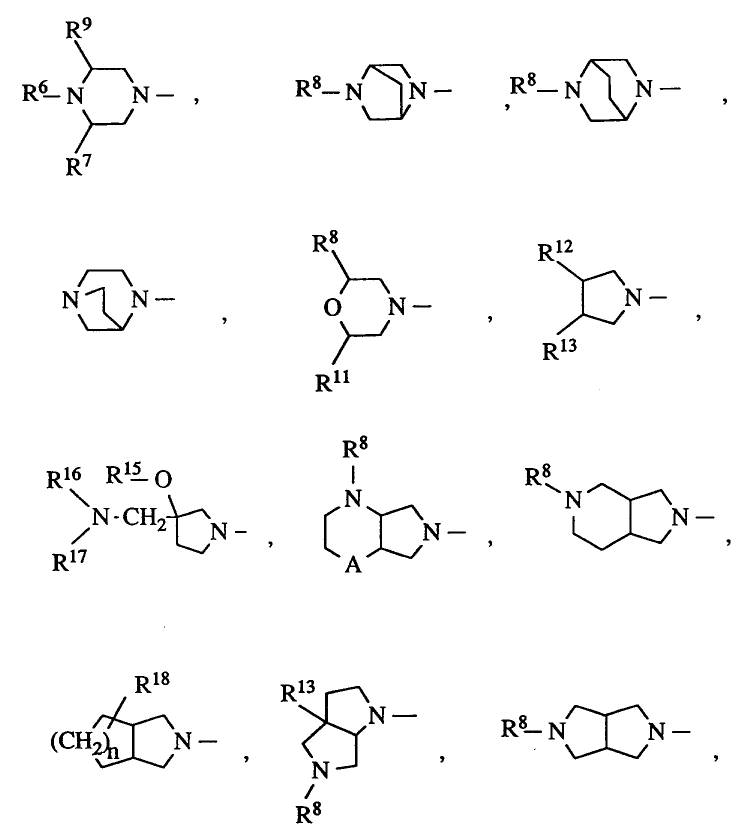 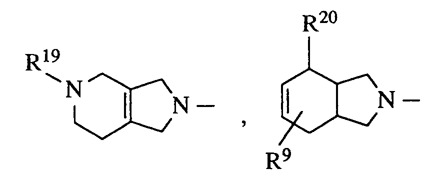 oder 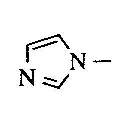 steht,
worin
- R⁶: für Wasserstoff, Methyl, gegebenenfalls durch Hydroxy substituiertes Ethyl,
- R⁷: für Wasserstoff oder Methyl,
- R⁸: für Wasserstoff oder Methyl,
- R⁹: für Wasserstoff oder Methyl,
- R¹¹: für Wasserstoff oder -CH₂-NH₂,
- R¹²: für Wasserstoff, Methyl, Amino, Methylamino, Aminomethyl oder Ethylaminomethyl,
- R¹³: für Wasserstoff, Hydroxy oder Methoxy,
- R¹⁵: für Wasserstoff oder Methyl,
- R¹⁶: für Wasserstoff oder Methyl,
- R¹⁷: für Wasserstoff oder Methyl,
- R¹⁸: für 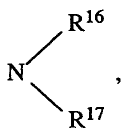
- R¹⁹: für Wasserstoff oder Methyl,
- R²⁰: für 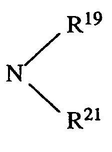 steht,
worin
- R²¹: Wasserstoff oder Methyl bedeutet,
- A: für CH₂, O oder für eine direkte Bindung steht und
- n: für 1 steht.

Weiterhin wurde gefunden, daß man die Verbindungen der Formel (I) erhält, wenn man eine Verbindung der Formel (II) 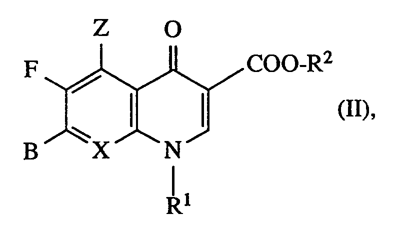
in welcher
- R¹, R², X und Z: die oben angegebenen Bedeutungen haben und
- B: für Halogen, insbesondere Fluor oder Chlor, steht,
mit Verbindungen der Formel (III)

Y-H (III),

in welcher
- Y: die oben angegebene Bedeutung hat,
gegebenenfalls in Gegenwart von Säurefängern umsetzt.

Verwendet man beispielsweise 1-Cyclopropyl-5-vinyl-6,7,8-trifluor-1,4-dihydro-4-oxo-3-chinolincarbonsäure und 1-Methylpiperazin als Ausgangsverbindungen, so kann der Reaktionsverlauf durch das folgende Formelschema wiedergegeben werden: 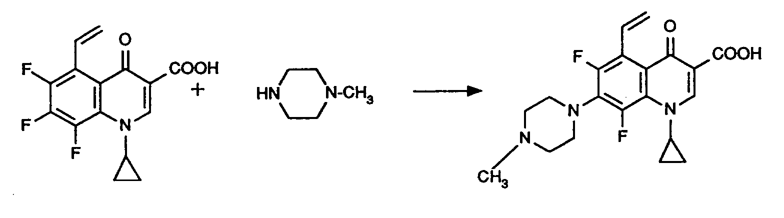
Weiterhin wurde gefunden, daß man Verbindungen der Formel (I) erhält, wenn man eine Verbindung der Formel (IV) 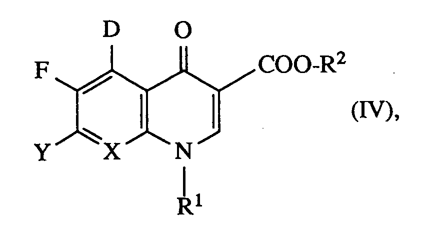
in welcher
- R¹, R², X und Y: die oben angegebenen Bedeutungen haben und
- D: für Halogen, insbesondere Chlor, Brom oder Iod oder eine Grupe OSO₂R_{F} steht, wobei R_{F} für einen Perfluoralkylrest mit 1 bis 8 C-Atomen steht,
mit metallorganischen Vinyl- oder Alkinylverbindungen der Formel (V)

M-Z (V),

in welcher
- Z: die oben angegebene Bedeutung hat und
- M: für SnR'₃, ZnX', B(OR'')₂,
worin
- R': C₁-C₄-Alkyl,
- R'': Wasserstoff oder C₁-C₄-Alkyl und
- X': Brom oder Chlor bedeutet,
steht,
in Gegenwart von Übergangsmetall-Katalysatoren umsetzt und gegebenenfalls vorhandene Schutzgruppen abspaltet.

Verwendet man beispielsweise 5-Brom-1-cyclopropyl-6,8-difluor-1,4-dihydro-7-(4-methyl-1,4-piperazin-1-yl)-4-oxo-3-chinolincarbonsäure und Tributylstannyltrimethylsilyl-acetylen als Ausgangsverbindungen, so kann der Reaktionsverlauf durch das folgende Formelschema wiedergegeben werden: 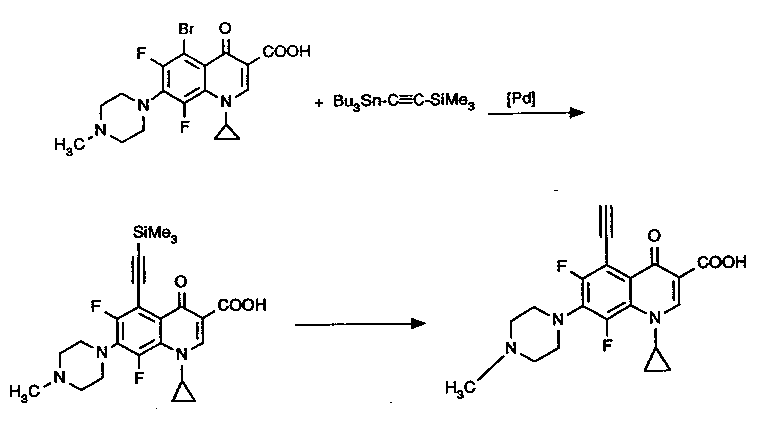
Die als Ausgangsverbindungen verwendeten Verbindungen der Formel (II) sind neu. Sie können hergestellt werden, indem man Chinolincarbonsäure-Derivate der Formel (VI) 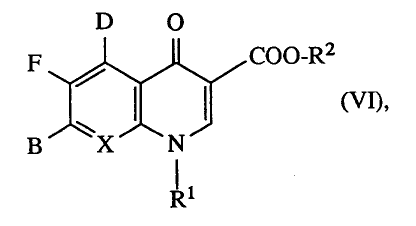
in welcher
- R¹, R², X und B: die oben angegebenen Bedeutungen haben und
- D: für Halogen, insbesondere Jod, Brom oder Chlor, oder eine Gruppe OSO₂R_{F} steht, wobei R_{F} für einen Perfluoralkylrest mit 1 bis 8 C-Atomen steht,
mit metallorganischen Vinyl- oder Alkinylverbindungen der Formel (V)

M-Z (V),

in welcher
- M und Z: die oben angegebenen Bedeutungen haben,
in Gegenwart von Übergangsmetall-Katalysatoren umsetzt und gegebenenfalls vorhandene Schutzgruppen abspaltet.

Verwendet man beispielsweise Vinyl-tributylzinn und 5-Brom-1-cyclopropyl-6,7,8-trifluor-1,4-dihydro-4-oxo-3-chinolincarbonsäure-ethylester als Ausgangsverbindungen, so kann der Reaktionsverlauf durch das folgende Formelschema wiedergegeben werden: 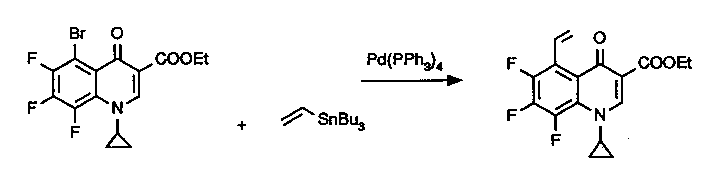
Die Komplexkatalysatoren werden in Anteilen von 0,1 bis 20 mol-%, bezogen auf eingesetzten 5-Halogen-chinoloncarbonsäureester, eingesetzt; bevorzugt sind Anteile von 0,5 bis 10 mol-%, ganz besonders bevorzugt Anteile von 1 bis 5 mol-%.

Die Kupplungsreaktionen werden in geeigneten inerten Lösungsmitteln wie beispielsweise Benzol, Toluol, Xylol, Dimethylformamid, Dimethylacetamid, Dimethoxyethan oder Gemischen solcher Lösungsmittel durchgeführt; bevorzugt sind Dimethylformamid und Toluol. Die Lösungsmittel werden vor Gebrauch nach bekannten Verfahren getrocknet und luftfrei gemacht.

Die Kupplungsreaktionen werden bei Temperaturen zwischen 20 und 200°C durchgeführt; bevorzugt sind Temperaturen zwischen 50 und 180°C.

Die Dauer der Umsetzung richtet sich nach der Reaktivität der Edukte und beträgt im allgemeinen zwischen 2 und 40 Stunden; bevorzugt sind Reaktionszeiten zwischen 4 und 24 Stunden.

Die Umsetzungen werden unter einer Schutzgasatmosphäre durchgeführt. Als Schutzgas kommen inerte Gase wie beispielsweise Helium, Argon oder Stickstoff in Frage; bevorzugt ist Stickstoff. Die Kupplungsreaktion wird im allgemeinen bei Normaldruck durchgeführt. Es ist jedoch selbstverständlich auch möglich, die Reaktion bei vermindertem oder erhöhtem Druck durchzuführen.

Die für die Kupplungsreaktion benötigten metallorganischen Vinyl- und Alkinyl-verbindungen sind entweder bekannt oder können nach literaturbekannten Methoden synthetisiert werden. So lassen sich zum Beispiel Vinyl-trialkylzinn-Verbindungen aus den entsprechenden Vinyliodiden, -bromiden oder -chloriden dadurch herstellen, daß man durch Umsetzung mit Magnesium die Vinylgrignard-Verbindungen gewinnt und diese mit einem Trialkylzinnchlorid zu den gewünschten Vinylzinn-Derivaten reagieren läßt.

Metallorganische Alkinylverbindungen lassen sich in bekannter Weise zum Beispiel dadurch darstellen, daß man das 1-Alkin bei Temperaturen zwischen -20 und -78°C in einem aprotischen Lösungsmittel wie zum Beispiel Tetrahydrofuran mit n-Butyl-, sec-Butyl- oder tert-Butyllithium metalliert und anschießend mit einer Metallhalogen-Verbindung wie zum Beispiel Zinkchlorid, Magnesiumbromid, Kupferiodid oder Trialkylzinnchlorid umsetzt. Bevorzugt ist die Umsetzung bei -78°C. Außer dem bevorzugten Lösungsmittel Tetrahydrofuran sind auch andere Ether wie Diethylether, Dipropylether oder tert-Butyl-methyl-ether oder Mischungen solcher Ether mit aprotischen aliphatischen oder aromatischen Lösungsmitteln wie n-Hexan oder Toluol möglich. Sowohl bei den Vinyl- als auch bei den Alkinylderivaten sind die Zinkchlorid- und Trialkylzinn-Derivate bevorzugt. Unter "Alkyl" wird bei den Trialkylzinnverbindungen C₁- bis C₆-Alkyl verstanden; bevorzugt sind Methyl und n-Butyl.

Trialkylvinylzinn-Verbindungen können nach literaturbekannten Methoden auch durch Hydrostannylierung von Alkinen mit Trialkylzinnhydriden in Gegenwart von Übergangsmetallkatalysatoren erhalten werden.

Die metallorganischen Vinyl- und Alkinylverbindungen werden mit 5-Halogen-chinoloncarbonsäure-Derivaten der allgemeinen Formel (IV) in Gegenwart eines geeigneten Katalysators nach im Prinzip bekannten Verfahren umgesetzt. "Halogen" steht hierbei für Jod, Brom oder Chlor; bevorzugt sind Brom und Chlor, besonders bevorzugt ist Brom.

Als Katalysatoren kommen beispielsweise Übergangsmetallverbindungen der Metalle Kobalt, Ruthernium, Rhodium, Iridium, Nickel, Palladium oder Platin in Frage. Bevorzugt sind Verbindungen der Metalle Platin, Palladium und Nickel, besonders bevorzugt ist Palladium. Solche Übergangsmetalle können in Form ihrer Salze wie zum Beispiel als NiCl₂, PdCl₂ oder Pd(OAc)₂, oder in Form von Komplexen mit geeigneten Liganden eingesetzt werden. Bevorzugt ist die Verwendung von Komplexen. Als Liganden werden bevorzugt Phosphine wie zum Beispiel Triphenylphosphin, Tri(o-tolyl)phosphin, Trimethylphosphin, Tributylphosphin und Tri(2-furyl)phosphin eingesetzt, bevorzugt ist Triphenylphosphin. Als bevorzugte Komplexkatalysatoren seien Bis(triphenylphosphin)nickel(II)-chlorid, Bis(triphenylphosphin)palladium(II)-chlorid, Tris(triphenylphosphin)palladium(0) und Tetrakis(triphenylphosphin)palladium(0) genannt.

Die als Ausgangsverbindungen verwendeten Verbindungen der Formel (IV) sind bekannt oder können nach bekannten Verfahren hergestellt werden.

Die als Ausgangsverbindungen verwendeten Amine der Formel (III) sind zum größten Teil bekannt. Chirale Amine können sowohl als Racemate als auch als enantiomerenreine oder diastereomerenreine Verbindungen eingesetzt werden. Als Beispiele seien genannt:
Piperazin,
1-Methylpiperazin,
1-Ethylpiperazin,
1-(2-Hydroxyethyl)-piperazin,
3-Methylpiperazin,
cis-2,6-Dimethyl-piperazin,
cis-2,3-Dimethyl-piperazin,
1,2-Dimethylpiperazin,
1-Cyclopropyl-piperazin,
2-Phenyl-piperazin,
2-(4-Pyridyl)-piperazin,
2-(2-Thienyl)-piperazin,
1,4-Diazabicyclo[3.2.1]octan,
8-Methyl-3,8-diazabicyclo[3.2.1]octan-Dihydrochlorid,
3-Methyl-3,8-diazabicyclo[3.2.1]octan-Dihydrochlorid,
2,5-Diazabicyclo[2.2.1]heptan-Dihydrochlorid,
2-Methyl-2,5-diazabicyclo[2.2.1]heptan-Dihydrochlorid,
2,5-Diazabicyclo[2.2.2]octan-Dihydrochlorid,
2-Methyl-2,5-diazabicyclo[2.2.2]octan-Dihydrochlorid,
1,4-Diazabicyclo[3.1.1]heptan,
Morpholin,
2,6-Dimethyl-morpholin,
2-Aminomethyl-morpholin,
2-tert.-Butoxycarbonylaminomethyl-morpholin,
2-Methylaminomethyl-morpholin,
2-Dimethylaminomethyl-morpholin,
Imidazol,
4-Methyl-imidazol,
Pyrrol,
3-Aminomethyl-2,5-dihydro-pyrrol,
3-Aminomethyl-4-methyl-2,5-dihydro-pyrrol,
3-(1-Aminoethyl)-2,5-dihydro-pyrrol,
3-Amino-azetidin,
3-tert.-Butoxycarbonylamino-azetidin,
3-tert.-Butoxycarbonylamino-2-methyl-azetidin,
3-tert.-Butoxycarbonylamino-3-methyl-azetidin,
3-tert.-Butoxycarbonylaminomethyl-azetidin,
Pyrrolidin,
3-Amino-pyrrolidin,
3-tert.-Butoxycarbonylamino-pyrrolidin,
3-(2,2-Dimethyl-propylidenamino)-pyrrolidin,
3-Methylamino-pyrrolidin,
3-Dimethylamino-pyrrolidin,
3-Aminomethyl-pyrrolidin,
3-tert.-Butoxycarbonylaminomethyl-pyrrolidin,
4-Chlor-3-tert-butoxycarbonylaminomethyl-pyrrolidin,
3-tert.-Butoxycarbonylaminomethyl-3-methyl-pyrrolidin,
3-tert.-Butoxycarbonylamino-4-methyl-pyrrolidin,
3-tert.-Butoxycarbonylaminomethyl-3-methoxy-pyrrolidin,
3-Methylaminomethyl-pyrrolidin,
3-Ethylaminomethyl-pyrrolidin,
4-tert.-Butoxycarbonylamino-2-methyl-pyrrolidin,
2-Methyl-3-methylamino-pyrrolidin,
2-Methyl-4-methylamino-pyrrolidin,
3-(2-Hydroxyethylamino)-pyrrolidin,
3-Hydroxy-pyrrolidin,
3-Hydroxymethyl-pyrrolidin,
4-Amino-3-hydroxy-pyrrolidin,
3-Hydroxy-4-methylamino-pyrrolidin,
3-tert.-Butoxycarbonylamino-4-methoxy-pyrrolidin,
3-Methylaminomethyl-3-hydroxy-pyrrolidin,
3-Dimethylaminomethyl-3-hydroxy-pyrrolidin,
3-Diethylaminomethyl-3-hydroxy-pyrrolidin,
3-tert.-Butylaminomethyl-3-hydroxy-pyrrolidin,
3-Methylamino-4-hydroxymethyl-pyrrolidin,
4-Methoxy-3-methylamino-pyrrolidin,
3-Methoxy-3-methylaminomethyl-pyrrolidin,
3-Amino-4-methoxy-2-methyl-pyrrolidin,
3-tert.-Butoxycarbonylamino-3-methyl-pyrrolidin,
3-Methyl-4-tert.-butoxycarbonylaminomethyl-pyrrolidin,
3-Methoxy-4-tert.-butoxycarbonylaminomethyl-pyrrolidin,
3-(1-Imidazolyl)-pyrrolidin,
6-Hydroxy-3-azabicyclo[3.3.0]octan,
6-Amino-3-azabicyclo[3.3.0]octan,
1-Amino-3-azabicyclo[3.3.0]octan,
1-Aminomethyl-3-azabicyclo[3.3.0]octan,
1-Ethylaminomethyl-3-azabicyclo[3.3.0]octan,
6-Amino-3-azabicyclo[4.3.0]nonan,
3-Amino-4-methylen-pyrrolidin,
7-Amino-5-azaspiro[2.4]heptan,
3,7-Diazabicyclo[3.3.0]octan,
3-Methyl-3,7-diazabicyclo[3.3.0]octan,
2,8-Diazabicyclo[4.3.0]nonan,
2-Methyl-2,8-diazabicyclo[4.3.0]nonan,
3-Methyl-3,8-diazabicyclo[4.3.0]nonan,
2-Oxa-5,8-diazabicyclo[4.3.0]nonan,
5-Methyl-2-oxa-5,8-diazabicyclo[4.3.0]nonan,
2,7-Diazabicyclo[3.3.0]octan,
2-Methyl-2,7-diazabicyclo[3.3.0]octan,
3-Methyl-2,7-diazabicyclo[3.3.0]octan,
4-Methyl-2,7-diazabicyclo[3.3.0]octan,
5-Methyl-2,7-diazabicyclo[3.3.0]octan-7-carbonsäure-tert-butylester,
7-Methyl-2,7-diazabicyclo[3.3.0]octan,
8-Methyl-2,7-diazabicyclo[3.3.0]octan,
7,8-Dimethyl-2,7-diazabicyclo[3.3.0]octan,
2,3-Dimethyl-2,7-diazabicyclo[3.3.0]octan,
2,8-Dimethyl-2,7-diazabicyclo[3.3.0]octan,
1,4-Diazatricyclo[6.2.0.0^{2,6}]decan,
1,4-Diazatricyclo[6.3.0.0^{2,6}]undecan,
2,7-Diazaspiro[4.4]nonan,
2-Methyl-2,7-diazaspiro[4.4]nonan,
4-Amino-1,3,3a,4,7,7a-hexahydroisoindol,
4-Methylamino-1,3,3a,4,7,7a-hexahydroisoindol,
5-Methyl-4-methylamino-1,3,3a,4,7,7a-hexahydroisoindol`
6-Methyl-4-methylamino-1,3,3a,4,7,7a-hexahydroisoindol,
7-Methyl-4-methylamino-1,3,3a,4,7,7a-hexahydroisoindol,
7a-Methyl-4-methylamino-1,3,3a,4,7,7a-hexahydroisoindol,
6,7-Dimethyl-4-methylamino-1,3,3a,4,7,7a-hexahydroisoindol,
4-Dimethylamino-1,3,3a,4,7,7a-hexahydroisoindol,
4-Ethylamino-1,3,3a,4,7,7a-hexahydroisoindol,
4-Aminomethyl-1,3,3a,4,7,7a-hexahydroisoindol,
4-Methylaminomethyl-1,3,3a,4,7,7a-hexahydroisoindol,
4-Hydroxy-1,3,3a,4,7,7a-hexahydroisoindol,
2,3,4,5,6,7-Hexahydro-1H-pyrrolo[3,4-c]pyridin,
5-Methyl-2,3,4,5,6,7-hexahydro-1H-pyrrolo[3,4-c]pyridin,
5-Ethyl-2,3,4,5,6,7-hexahydro-1H-pyrrolo(3,4-c]pyridin,
5-(tert.-Butoxycarbonyl)-2,3,4,5,6,7-hexahydro-1H-pyrrolo[3,4-c]pyridin.

Die Umsetzung von (II) mit (III), bei der die Verbindungen (III) auch in Form ihrer Salze, wie z.B. der Hydrochloride eingesetzt werden können, wird vorzugsweise in einem Verdünnungsmittel wie Dimethylsulfoxid, N,N-Dimethylformamid, N-Methylpyrrolidon, Hexamethyl-phosphorsäuretrisamid, Sulfolan, Acetonitril, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo[2.2.2]octan (DABCO), 1,8-Diazabicyclo[5.4.0]undec-7-en (DBU) oder überschüssiges Amin (III).

Die Reaktionstemperaturen können in einem größeren Bereich variiert werden. Im allgemeinen arbeitet man zwischen etwa 20 und 200°C, vorzugsweise zwischen 80 und 180°C.

Die Umsetzung kann bei Normaldruck, aber auch bei erhöhtem Druck durchgeführt werden. Im allgemeinen arbeitet man bei Drucken zwischen etwa 1 und 100 bar, vorzugsweise zwischen 1 und 10 bar.

Bei der Durchführung des erfindungsgemäßen Verfahrens setzt man auf 1 Mol der Verbindung (II) 1 bis 15 Mol, vorzugsweise 1 bis 6 Mol der Verbindung (III) ein.

Freie Aminogruppen können wahrend der Umsetzung durch eine geeignete Aminoschutzgruppe, zum Beispiel durch den tert.-Butoxycarbonylrest, geschützt und nach Beendigung der Reaktion durch Behandlung mit einer geeigneten Säure wie Chlorwasserstoffsäure oder Trifluoressigsäure wieder freigesetzt werden.

Die erfindungsgemäßen Ester werden durch Umsetzung eines Alkalisalzes der zugrundeliegenden Carbonsäure, die gegebenenfalls am N-Atom durch eine Schutzgruppe wie den tert.-Butoxycarbonylrest geschützt sein kann, mit geeigneten Halogenalkylderivaten in einem Lösungsmittel wie Dimethylformamid, Dimethylacetamid, N-Methylpyrrolidon, Dimethylsulfoxid oder Tetramethylharnstoff bei Temperaturen von etwa 0 bis 100°C, vorzugsweise 0 bis 50°C, erhalten.

Die Herstellung der Säureadditionssalze der erfindungsgemäßen Verbindungen erfolgt in üblicher Weise zum Beispiel durch Lösen des Betains in ausreichender Menge wäßriger Säure und Ausfällen des Salzes mit einem mit Wasser mischbaren organischen Lösungsmittel wie Methanol, Ethanol, Aceton, Acetonitril. Man kann auch äquivalente Mengen Betain und Säure in Wasser oder einem Alkohol wie Glykolmonomethylether erhitzen und anschließend bis zur Trockne eindampfen oder das ausgefallene Salz absaugen. Als pharmazeutisch verwendbare Salze sind beispielsweise die Salze der Salzsäure, Schwefelsäure, Essigsäure, Glykolsäure, Milchsäure, Bernsteinsäure, Zitronensäure, Weinsäure, Methansulfonsäure, 4-Toluolsulfonsäure, Galacturonsäure, Gluconsäure, Embonsäure, Glutaminsäure oder Asparaginsäure zu verstehen.

Die Alkali- oder Erdalkalisalze der erfindungsgemäßen Carbonsäuren werden beispielsweise durch Lösen des Betains in unterschüssiger Alkali- oder Erdalkalilauge, Filtration von ungelöstem Betain und Eindampfen des Filtrats bis zur Trockne erhalten. Pharmazeutisch geeignet sind Natrium-, Kalium- oder Calciumsalze. Durch Umsetzung eines Alkali- oder Erdalkalisalzes mit einem geeigneten Silbersalz wie Silbernitrat werden die entsprechenden Silbersalze erhalten.

Außer den in den Beispielen genannten Wirkstoffen können auch die in der folgenden Tabelle aufgeführten Wirkstoffe hergestellt werden:

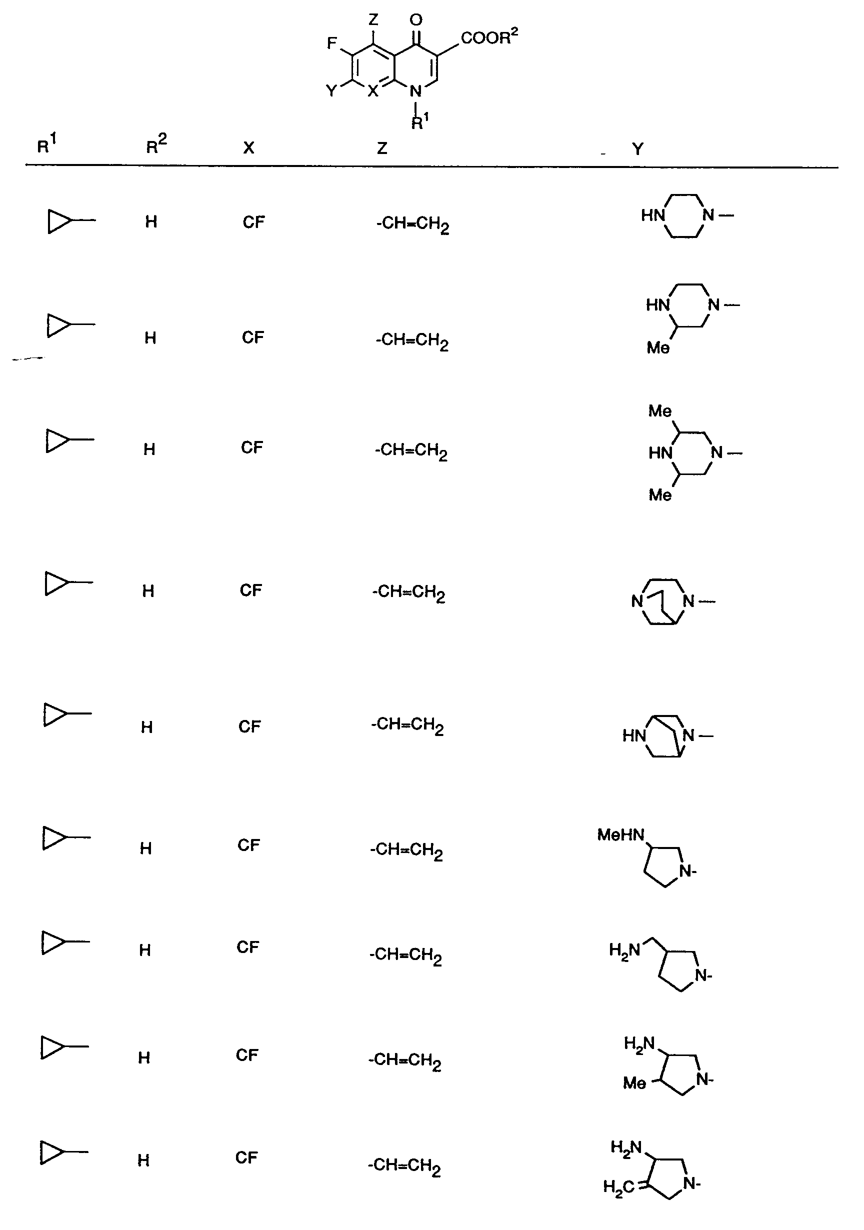

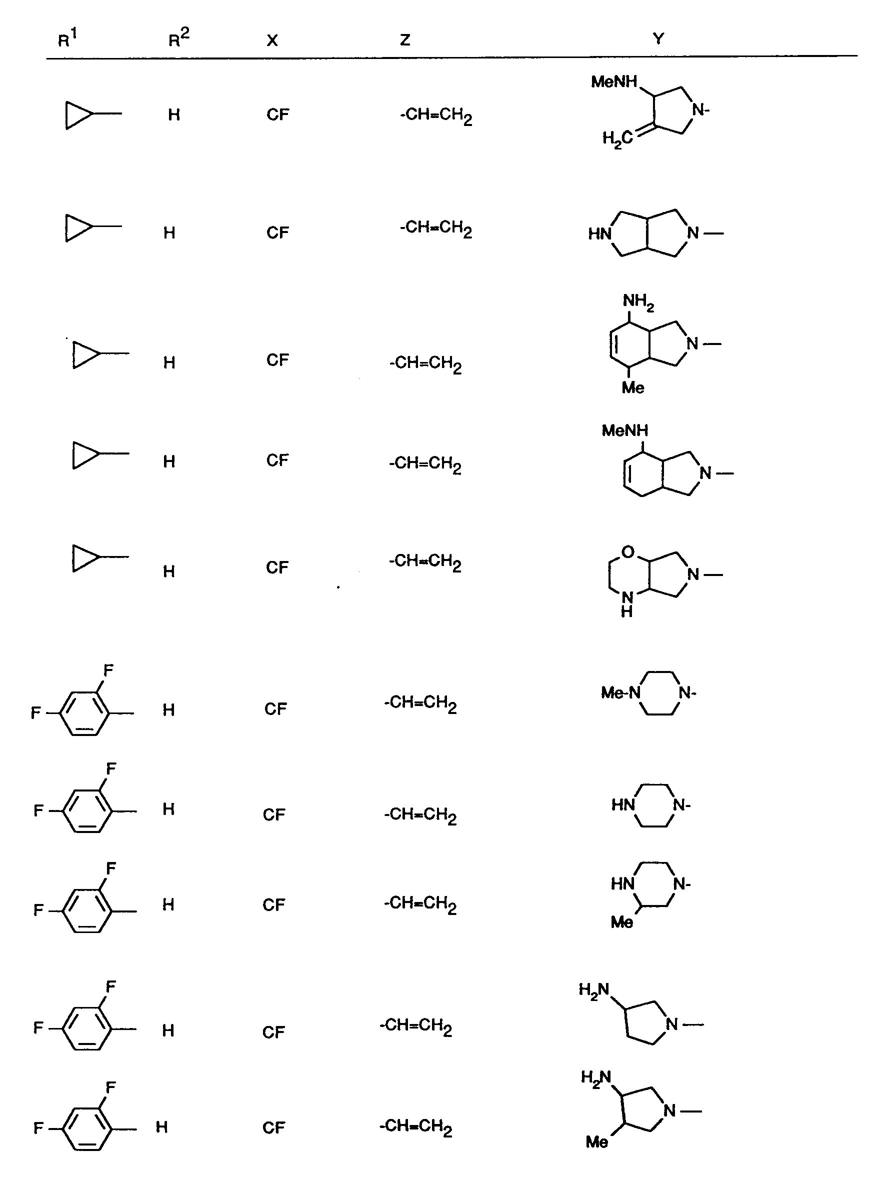

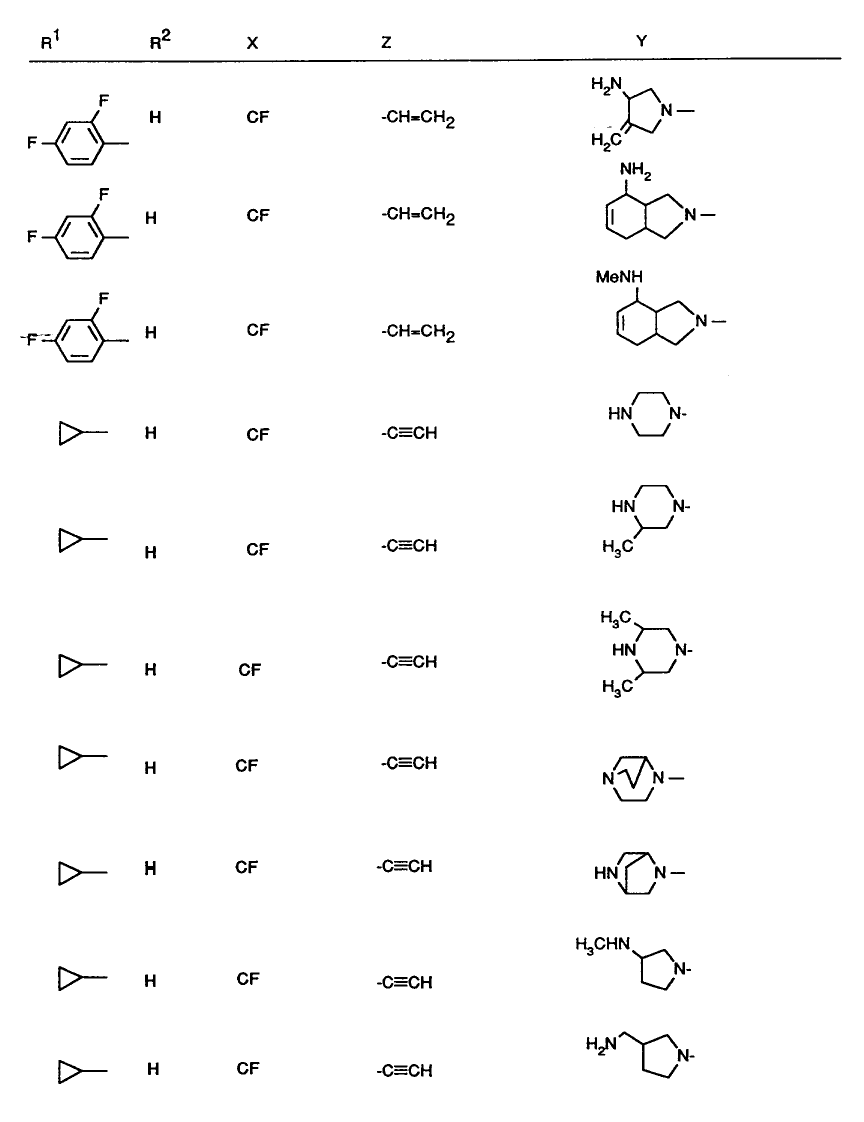

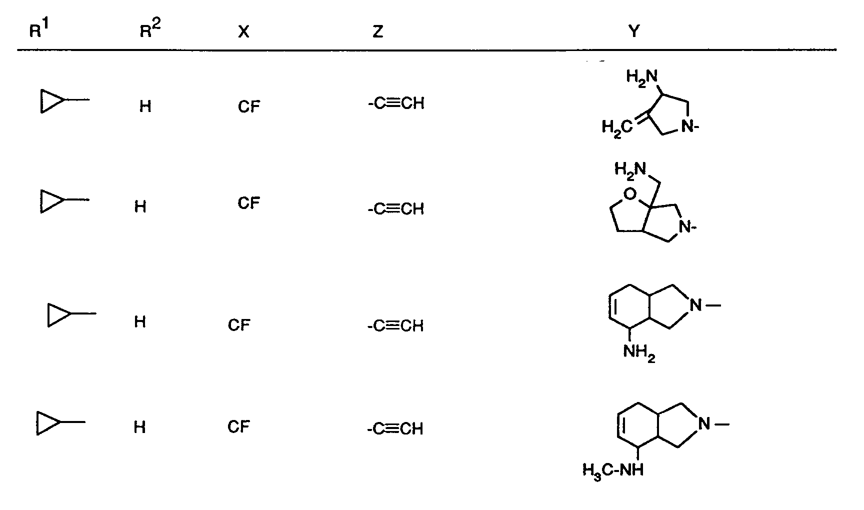

Die erfindungsgemäßen Verbindungen wirken stark antibiotisch und zeigen bei geringer Toxizität ein breites antibakterielles Spektrum gegen grampositive und gramnegative Keime, insbesondere gegen Enterobakterien; vor allem auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gramnegative und grampositive Bakterien und bakterienähnliche Mikroorganismen bekämpft sowie die durch diese Erreger hervorgerufenen Erkrankungen verhindert, gebessert und/oder geheilt werden.

Die erfindungsgemäßen Verbindungen zeichnen sich durch verstärkte Wirkung auf ruhende und resistente Keime aus. Bei ruhenden Bakterien, also Bakterien, die kein nachweisbares Wachstum zeigen, wirken die Verbindungen weit unterhalb von Konzentrationen bisher bekannter Substanzen. Dies bezieht sich nicht nur auf die einzusetzende Menge, sondern auch auf die Geschwindigkeit der Abtötung. Solche Ergebnisse konnten bei grampositiven und -negativen Bakterien, insbesondere bei Staphylococcus aureus, Pseudomonas aeruginosa, Enterococcus faecalis und Escherichia coli beobachtet werden.

Auch gegenüber Bakterien, die gegenüber vergleichbaren Substanzen als weniger empfindlich eingestuft werden, insbesondere resistenten Staphylococcus aureus, Escherichia coli, Pseudomonas aeruginosa und Enterococcus faecalis zeigen die erfindungsgemäßen Verbindungen überraschende Wirkungssteigerungen.

Auf Grund der hohen Wirksamkeit der erfindungsgemäßen Verbindungen gegen Bakterien und bakterienähnliche Mikroorganismen sind sie besonders gut zur Prophylaxe und Chemotherapie von lokalen und systemischen Infektionen in der Human- und Tiermedizin geeignet, die durch diese Erreger hervorgerufen werden.

Besonders wirksam sind die erfindungsgemäßen Verbindungen gegen typische und atypische Mykobakterien und Helicobacter pylori sowie gegen bakterienähnliche Mikroorganismen, wie zum Beispiel Mykoplasmen und Rickettsien.

Die Verbindungen eignen sich ferner zur Bekämpfung von Protozoonosen und Helminthosen.

Die erfindungsgemäßen Verbindungen können in verschiedenen pharmazeutischen Zubereitungen angewendet werden. Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Die minimalen Hemmkonzentrationen (MHK) wurden per Reihenverdünnungsverfahren auf Iso-Sensitest Agar (Oxoid) bestimmt. Für jede Prüfungssubstanz wurde eine Reihe von Agarplatten hergestellt, die bei jeweils doppelter Verdünnung abfallende Konzentrationen des Wirkstoffs enthielten. Die Agarplatten wurden mit einem Multipoint-Inokulator (Denley) beimpft. Zum Beimpfen wurden Übernachtkulturen der Erreger verwandt, die zuvor so verdünnt wurden, daß jeder Impfpunkt ca.-10⁴ koloniebildende Partikel enthielt. Die beimpften Agarplatten wurden bei 37°C bebrütet, und das Keimwachstum wurde nach ca. 20 Stunden abgelesen. Der MHK-Wert (µg/ml) gibt die niedrigste Wirkstoffkonzentration an, bei der mit bloßem Auge kein Wachstum zu erkennen war.

In der Tabelle 2 sind die MHK-Werte einiger der erfindungsgemäßen Verbindungen im Vergleich zu der aus EP 287 951 bekannten Verbindung K (1-Cyclopropyl-5-ethyl-6,8-difluor-1,4-dihydro-7-(4-methyl-1-piperazinyl)-4-oxo-3-chinolincarbonsäure) angegeben, die die bessere Wirksamkeit der erfindungsgemäßen Verbindungen gegenüber einer vergleichbaren Chinoloncarbonsäure mit einem C₂-Substituenten in der 5-Position belegen.

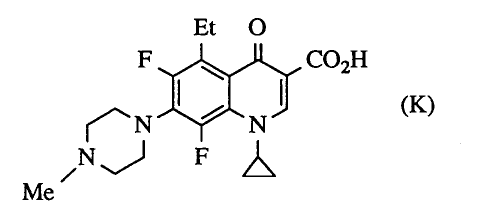

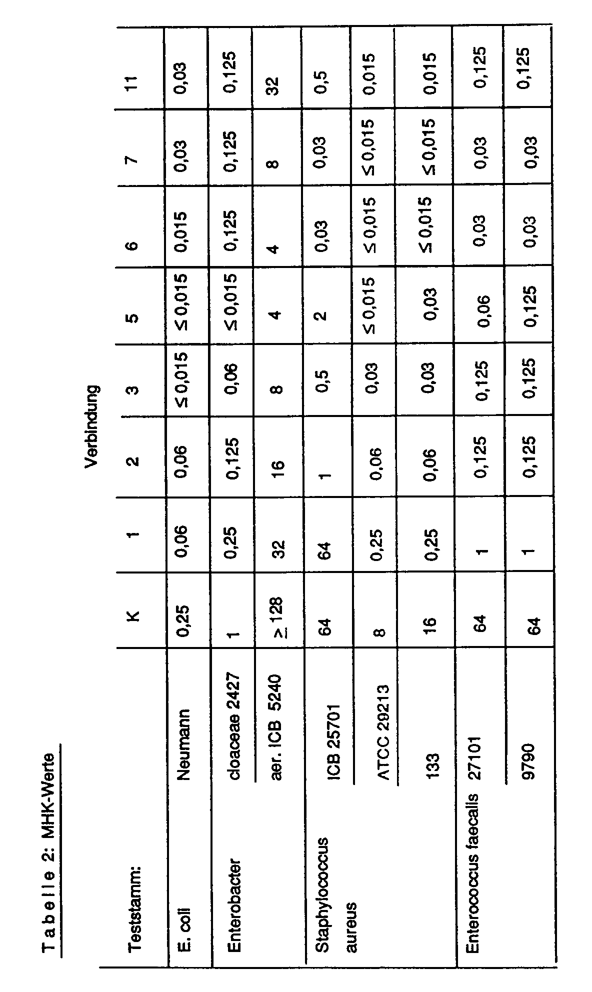

In der Tabelle 3 sind MHK-Werte erfindungsgemäßer Verbindungen gegen veterinärspezifische Keime aufgeführt. Als Vergleichsverbindung ist hier das Veterinärtherapeutikum Enrofloxacin (ENRO) angegeben.

**Tabelle 3**

| MHK-Werte | | | | |
|---|---|---|---|---|
| Teststamm | | ENRO | Verbindung | |
| | | | 5 | 6 |
| E. coli | G293 Lh | 32 | 32 | 8 |
| | 21 Bui | 32 | 16 | 4 |
| Staph. | Z433-4 LH | 0,25 | 0,03 | 0,015 |
| | Ky 469 We | 0,12 | 0,03 | 0,015 |
| | Z 318 Lh | 8 | 2 | 0,06 |

### Herstellung der Zwischenprodukte

### Beispiel A

### 1-Cyclopropyl-6,7,8-trifluor-1,4-dihydro-4-oxo-5-vinyl-3-chinolincarbonsäure-ethylester

5,9 g 5-Brom-1-cyclopropyl-6,7,8-trifluor-1,4-dihydro-4-oxo-3-chinolincarbonsäure-ethylester, 6,7 g Tributylvinylstannan und 0,69 g Tetrakis(triphenylphosphin)palladium(0) werden in 60 ml absolutem Toluol 10 Stunden unter einer Stickstoffatmosphäre zum Rückfluß erhitzt. Das Reaktionsgemisch wird auf -18°C gekühlt, der ausgefallene Feststoff abgesaugt, mit Toluol nachgewaschen und getrocknet. Man erhält 4,46 g der Titelverbindung (88 % der Theorie).
Schmelzpunkt: 188-189°C.

### Beispiel B

### 1-Cyclopropyl-6,7,8-trifluor-1,4-dihydro-4-oxo-5-vinyl-3-chinolincarbonsäure

3 g des Produkts aus Beispiel A werden in einem Gemisch aus 40 ml Eisessig, 2 ml Wasser und 0,6 ml konzentrierter Schwefelsäure 1,5 Stunden zum Rückfluß erhitzt. Danach wird das Reaktionsgemisch mit 40 ml Wasser versetzt und auf Raumtemperatur abgekühlt. Das Produkt wird abgesaugt, mit Wasser gewaschen und getrocknet. Es werden 2,08 g der Titelverbindung erhalten (75 % der Theorie).
Schmelzpunkt: 215-216°C.

### Beispiel C

### 1-(2,4-Difluorphenyl)-6,7,8-trifluor-1,4-dihydro-4-oxo-5-vinyl-3-chinolincarbonsäure-ethylester

4,6 g 5-Brom-1-(2,4-difluorphenyl)-6,7,8-trifluor-1,4-dihydro-4-oxo-3-chinolincarbonsäure-ethylester, 4,5 g Tributylvinylstannan und 0,46 g Tetrakis(triphenylphosphin)palladium(0) werden in 40 ml absolutem Toluol 10 Stunden unter einer Stickstoffatmosphäre zum Rückfluß erhitzt. Das Produkt wird bei Raumtemperatur abgesaugt, mit Wasser gewaschen und getrocknet Man erhält 2,7 g der Titelverbindung (66 % der Theorie).
Schmelzpunkt: 174-176°C.

### Beispiel D

### 1-(2,4-Difluorphenyl)-6,7,8-trifluor-1,4-dihydro-4-oxo-5-vinyl-3-chinolincarbonsäure

2 g des Produktes aus Beispiel C werden in einer Mischung aus 15 ml Eisessig, 1,5 ml Wasser und 0,7 ml konz. Schwefelsäure 2 Stunden zum Rückfluß erhitzt. Das Produkt wird bei Raumtemperatur abgesaugt mit Wasser gewaschen und getrocknet. Man erhält 1,3 g der Titelverbindung (71 % der Theorie).
Schmelzpunkt: 201-202°C.

### Beispiel E

### 1-Cyclopropyl-6,7,8-trifluor-1,4-dihydro-5-trimethylsilylethinyl-4-oxo-3-chinolincarbonsäure-ethylester

1,95 g 5-Brom-1-cyclopropyl-6,7,8-trifluor-1,4-dihydro-4-oxo-3-chinolincarbonsäure-ethylester, 2,7 g Tributylstannyl-trimethylsilyl-acetylen und 0,29 g Tetrakis-(triphenylphosphin)palladium(0) werden in 20 ml absolutem Toluol 4 Stunden unter einer Stickstoffatmosphäre zum Rückfluß erhitzt. Das Reaktionsgemisch wird bei Raumtemperatur filtriert; der Feststoff wird mit wenig Toluol gewaschen und getrocknet. Es werden 1,6 g der Titelverbindung erhalten (78 % der Theorie).
Schmelzpunkt: 267-269°C.

### Beispiel F

### 1-Cyclopropyl-5-ethinyl-6,7,8-trifluor-1,4-dihydro-4-oxo-3-chinolincarbonsäure-ethylester

1 g des Produktes aus Beispiel E und 0,45 g Kaliumfluorid werden in einer Mischung aus 20 ml Dimethylformamid, 10 ml Chloroform und 1 ml Wasser 1 Stunde bei Raumtemperatur verrührt. Nach Zugabe von Chloroform wird die organische Phase abgetrennt, mit Wasser ausgeschüttelt, über Natriumsulfat getrocknet und eingeengt. Der Rückstand wird mit 20 ml Methanol kurz aufgekocht. Nach Abkühlen auf 0°C wird der erhaltene Feststoff abgesaugt und getrocknet. Man erhält 0,63 g der Titelverbindung (77 % der Theorie).
Schmelzpunkt: 190°C (Zers.)

### Beispiel G

### 1-Cyclopropyl-6,8-difluor-1,4-dihydro-7-(4-methyl-1-piperazinyl)-5-trimethylsilyl-4 -oxo-3-chinolincarbonsäure-ethylester

1,1 g 5-Brom-1-cyclopropyl-6,8-difluor-1,4-dihydro-7-(4-methyl-1-piperazinyl)-4-oxo-3-chinolincarbonsäure-ethylester, 1,2 g Tributylstannyl-trimethylsilyl-acetylen und 0,135 g Tetrakis(triphenylphosphin)palladium(0) werden in 10 ml absolutem Toluol 3 Stunden zum Rückfluß erhitzt. Das Reaktionsgemisch wird mit weiteren 5 ml Toluol versetzt und heiß filtriert. Aus dem Filtrat kristallisiert das Produkt aus. Nach Absaugen und Trocknen erhält man 0,63 g der Titelverbindung (56 % der Theorie).
Schmelzpunkt: 231-233°C.

### Herstellung der Wirkstoffe

### Beispiel 1

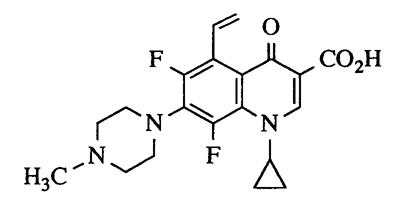

1,55 g (5 mmol) 1-Cyclopropyl-6,7,8-trifluor-1,4-dihydro-4-oxo-5-vinyl-3-chinolincarbonsäure werden in einer Mischung aus 10 ml Acetonitril und 5 ml Dimethylformamid mit 0,55 g (5 mmol) 1,4-Diazabicyclo[2.2.2]octan und 0,75 g (7,5 mmol) N-Methylpiperazin 1 Stunde unter Rückfluß erhitzt. Man engt ein, verrührt den Rückstand mit Wasser, saugt den ungelösten Niederschlag ab, wäscht mit Wasser und Acetonitril und trocknet bei 100°C im Hochvakuum.
Ausbeute: 1,0 g (51 % der Theorie) 1-Cyclopropyl-6,8-difluor-1,4-dihydro-4-oxo-7-(4-methyl-1-piperazinyl)-4-oxo-5-vinyl-3-chinolincarbonsäure.
Schmelzpunkt: 242-245°C (unter Zersetzung).

### Beispiel 2

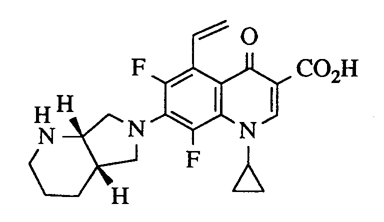

Analog Beispiel 1 setzt man mit cis-2,8-Diazabicyclo[4.3.0]nonan zu 1-Cyclopropyl-7-(cis-2,8-diazabicyclo[4.3.0]non-8-yl)-6,8-difluor-1,4-dihydro-4-oxo-5-vinyl-3-chinolincarbonsäure vom Schmelzpunkt 217-219°C (unter Zersetzung) (aus Glykolmonomethylether) um.

### Beispiel 3

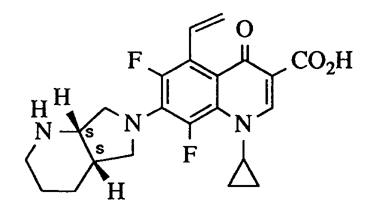

0,93 g 1-Cyclopropyl-6,7,8-trifluor-1,4-dihydro-4-oxo-5-vinyl-3-chinolincarbonsäure, 0,57 g S,S-2,8-Diazabicyclo[4.3.0]nonan und 0,34 g DABCO werden in einer Mischung aus 6 ml Acetonitril und 3 ml Dimethylformamid 2 Stunden zum Rückfluß erhitzt. Das Reaktionsgemisch wird im Vakuum eingeengt und der Rückstand mit 10 ml Wasser aufgekocht. Das Produkt wird heiß abgesaugt, mit Wasser gewaschen und getrocknet. Man erhält 0,76 g 1-Cyclopropyl-7-(S,S-2,8-diazabicyclo[4.3.0]non-8-yl)-6,8-difluor -1,4-dihydro-4-oxo-5-vinyl-3-chinolincarbonsäure.
Schmelzpunkt: 195-197°C (unter Zersetzung).

### Beispiel 4

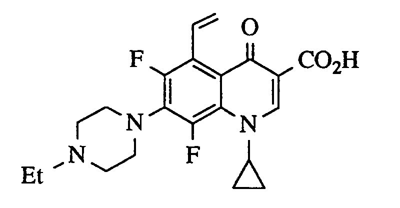

0,93 g 1-Cyclopropyl-6,7,8-trifluor-1,4-dihydro-4-oxo-5-vinyl-3-chinolincarbonsäure, 0,51 g 4-Ethyl-1,4-piperazin und 0,34 g DABCO werden 2,5 Stunden in einer Mischung aus 6 ml Acetonitril und 3 ml Dimethylformamid zum Rückfluß erhitzt. Das Reaktionsgemisch wird bei Raumtemperatur mit 15 ml Wasser versetzt, das Produkt abgesaugt, mit Wasser gewaschen und getrocknet. Man erhält 0,79 g 1-Cyclopropyl-7-(4-ethyl-1-piperazinyl)-6,8-difluor-1,4-dihydro-4-oxo-5-vinyl-3-chinolincarbonsäure.
Schmelzpunkt: 198-200°C

### Beispiel 5

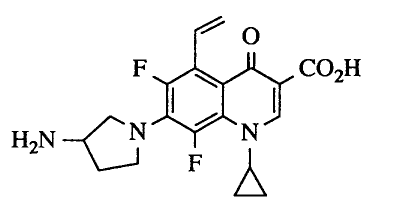

A) Eine Lösung von 0,39 g 3-Amino-pyrrolidin in 4 ml Acetonitril wird mit einer Lösung von 0,75 g 3-Nitrobenzaldehyd in 4 ml Acetonitril versetzt. Nach 1 Stunde bei Raumtemperatur wird das Reaktionsgemisch mit einer Lösung von 1 g DABCO und 0,93 g 1-Cyclopropyl-6,7,8-trifluor-1,4-dihydro-4-oxo-5-vinyl-3-chinolincarbonsäure in 4 ml Dimethylformamid versetzt. Nach 1 Stunde unter Rückfluß wird das Reaktionsgemisch auf 0°C abgekühlt, das Produkt abgesaugt und getrocknet. Man erhält 1,33 g 1-Cyclopropyl-6,8-difluor-1,4-dihydro-7-(3-(3-nitro-benzylidenamino)-pyrrolidin-1-yl)-4-oxo-5-vinyl-3-chinolincarbonsäure.
   Schmelzpunkt: 193-195°C (unter Zersetzung).
B) 1 g des Produktes aus Teil A) der Vorschrift wird in einer Mischung aus 22 ml Methylenchlorid und 30 ml 3N wäßriger Salzsäure 1,5 Stunden zum Rückfluß erhitzt. Nach Abkühlen auf 0°C wird das Produkt abgesaugt, mit Acetonitril gewaschen und getrocknet. Es werden 0,66 g 7-(3-Amino-pyrrolidin-1-yl)-1-cyclopropyl-6,8-difluor-1,4-dihydro-4-oxo-5-vinyl-3-chinolincarbonsäure Hydrochlorid erhalten.
   Schmelzpunkt: >300°C

### Beispiel 6

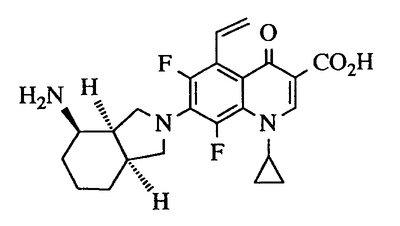

0,93 g 1-Cyclopropyl-6,7,8-trifluor-1,4-dihydro-4-oxo-5-vinyl-3-chinolincarbonsäure, 0,62 g 4-Amino-1,3,3a,4,7,7a-hexahydro-isoindol und 0,34 g DABCO werden in einem Gemisch aus 6 ml Acetonitril und 3 ml Dimethylformamid 1 Stunde zum Rückfluß erhitzt. Das Reaktionsgemisch wird mit 20 ml Wasser versetzt, das Produkt abgesaugt, mit Wasser gewaschen und getrocknet. Man erhält 1 g 7-(4-Amino-1,3,3a,4,7,7a-hexahydro-isoindol-2-yl)-1-cyclopropyl-6,8-difluor-1,4-dihydro-4-oxo-5- vinyl-3-chinolincarbonsäure.
Schmelzpunkt: 224-227°C (unter Zersetzung)

### Beispiel 7

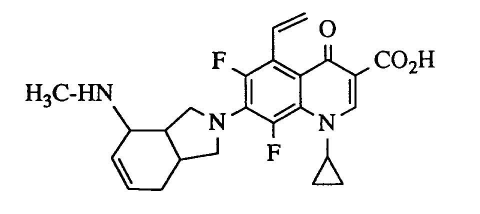

0,46 g 1-Cyclopropyl-6,7,8-trifluor-1,4-dihydro-4-oxo-5-vinyl-3-chinolincarbonsäure, 0,34 g 4-Methylamino-1,3,3a,4,7,7a-hexahydro-isoindol und 0,18 g DABCO werden in einem Gemisch aus 3 ml Acetonitril und 1,5 ml Dimethylformamid 2 Stunden zum Rückfluß erhitzt. Das Reaktionsgemisch wird bei Raumtemperatur mit 20 ml Wasser versetzt, das Produkt abgesaugt, mit Wasser gewaschen und getrocknet. Es werden 0,5 g 7-(4-Methylamino-1,3,3a,4,7,7a-hexahydro-isoindol-2-yl)-1-cyclopropyl-6,8-difluor-1,4-dihydro-4-oxo-5-vinyl-3-chinolincarbonsäure erhalten.
Schmelzpunkt: 206-208°C (unter Zersetzung)

### Beispiel 8

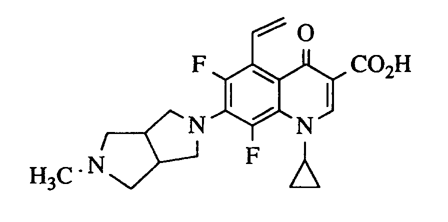

0,46 g 1-Cyclopropyl-6,7,8-trifluor-1,4-dihydro-4-oxo-5-vinyl-3-chinolincarbonsäure, 0,29 g 4-Methyl-octahydropyrrolo[3,4-c]pyrrol und 0,18 g DABCO werden in einem Gemisch aus 3 ml Acetonitril und 1,5 ml Dimethylformamid 2 Stunden zum Rückfluß erhitzt. Das Reaktionsgemisch wird anschließend eingeengt und der Rückstand mit 10 ml Wasser versetzt. Der Feststoff wird abgesaugt, mit Wasser gewaschen und getrocknet. Man erhält 0,5 g 1-Cyclopropyl-6,8-difluor-1,4-dihydro-7-(5-methyl-hexahydro-pyrrolo[3,4-c]pyrrol-2-yl)-4-oxo-5-vinyl-3-chinolincarbonsäure.
Schmelzpunkt: 156-157°C

### Beispiel 9

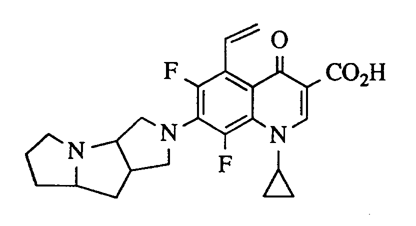

0,93 g 1-Cyclopropyl-6,7,8-trifluor-1,4-dihydro-4-oxo-5-vinyl-3-chinolincarbonsäure, 0,69 g Decahydropyrrolo[3,4-b]pyrrolizin und 0,36 g DABCO werden in einem Gemisch aus 6 ml Acetonitril und 3 ml Dimethylformamid 2 Stunden zum Rückfluß erhitzt. Das Reaktionsgemisch wird bei Raumtemperatur mit 20 ml Wasser versetzt, der Feststoff abgesaugt, mit Wasser gewaschen und getrocknet. Es werden 0,7 g 1-Cyclopropyl-6,8-difluor-1,4-dihydro-7-(octahydro-pyrrolo[3,4-b]pyrrolizin-2-yl)-4-oxo-5- vinyl-3-chinolincarbonsäure erhalten.
Schmelzpunkt: 154-156°C

### Beispiel 10

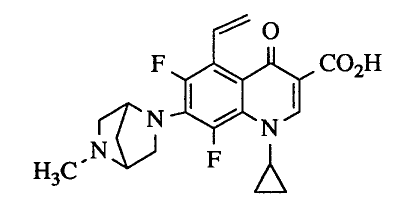

0,93 g 1-Cyclopropyl-6,7,8-trifluor-1,4-dihydro-4-oxo-5-vinyl-3-chinolincarbonsäure, 0,5 g 2-Methyl-2,5-diazabicyclo[2.2.1]heptan und 0,36 g DABCO werden in einem Gemisch aus 6 ml Acetonitril und 3 ml Dimethylformamid 2 Stunden zum Rückfluß erhitzt. Das Reaktionsgemisch wird bei Raumtemperatur mit 2 ml Wasser versetzt, der Feststoff abgesaugt, mit Wasser gewaschen und getrocknet. Man erhält 0,9 g 1-Cyclopropyl-6,8-difluor-1,4-dihydro-7-(2-methyl-2,5-diazabicyclo[2.2.1]hept-5-yl)-4-oxo-5- vinyl-3-chinolincarbonsäure.
Schmelzpunkt: 199-201°C

### Beispiel 11

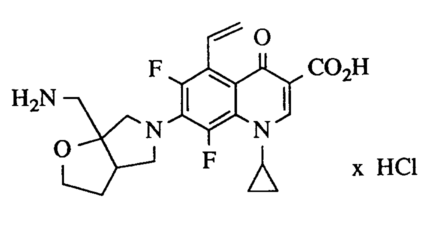

a) 0,46 g 1-Cyclopropyl-6,7,8-trifluor-1,4-dihydro-4-oxo-5-vinyl-3-chinolincarbonsäure, 0,55 g tert.-Butoxycarbonyl-(hexahydro-furo[2,3-c]pyrrol-6a-methyl)-amin und 0,18 g DABCO werden in einem Gemisch aus 3 ml Acetonitril und 1,5 ml Dimethylformamid 2 Stunden zum Rückfluß erhitzt. Das Reaktionsgemisch wird nach dem Abkühlen mit 10 ml Wasser versetzt, der Feststoff abgesaugt, mit Wasser gewaschen und getrocknet. Man erhält 0,6 g 1-Cyclopropyl-6,8-difluor-1,4-dihydro-4-oxo-7-(6a-tert.-butoxycarbonylaminomethyl-hexahydro-furo[2,3-c]pyrrol-5-yl)-5-vinyl-3-chinolincarbonsäure.
   Schmelzpunkt: 152-153°C (Zers.)
b) 0,53 g des Feststoffes aus Teil a) werden bei 50°C portionsweise in 5 ml 20 %ige wäßrige Salzsäure eingetragen. Nach 2 Stunden Rühren bei Raumtemperatur werden noch 2 ml konz. wäßrige Salzsäure zugegeben. Nach 2 Stunden bei Raumtemperatur wird das Reaktionsgemisch im Ölpumpenvakuum eingeengt. Der Rückstand wird mit 4 ml Ethanol verrührt, abgesaugt und getrocknet. Man erhält 0,33 g 7-(6a-Aminomethyl-hexahydro-furo[2,3-c]pyrrol-5-yl)-1-cyclopropyl-6,8-difluor-1,4-dihydro-4-oxo-5-vinyl-3-chinolincarbonsäure Hydrochlorid.
   Schmelzpunkt: 244-247°C (Zers.)

### Beispiel 12

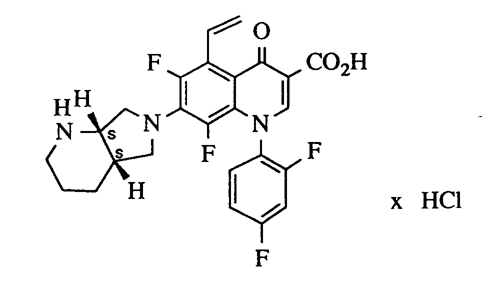

A) 381 mg (1 mmol) 1-(2,4-Difluorphenyl)-6,7,8-trifluor-1,4-dihydro-4-oxo-5-vinyl-3-chinolincarbonsäure werden in einer Mischung aus 2 ml Acetonitril und 1 ml Dimethylformamid suspendiert, mit 112 mg (1 mmol) 1,4-Diazabicyclo[2.2.2]-octan und 150 mg (1,2 mmol) S,S-2,8-Diazabicyclo[4.3.0]nonan versetzt und 1 Stunde unter Rückfluß erhitzt. Man engt die Mischung ein, verrührt den Rückstand mit Wasser, saugt den ungelösten Niederschlag ab, wäscht mit Wasser und kristallisiert aus Glykolmonomethylether um.
   Ausbeute: 250 mg (51 % der Theorie) 7-(S,S-2,8-Diazabicyclo[4.3.0]non-8-yl)-1-(2,4-difluorphenyl)-6,8-difluor-1,4-dihydro-4-oxo-5-vinyl-3-chinolincarbonsäure.
   Schmelzpunkt: 212-215°C (unter Zersetzung).
B) 150 mg (0,3 mmol) des Betains aus Stufe A werden in 15 ml halbkonzentrierter Salzsäure in der Hitze gelöst Die Lösung wird eingeengt, der Rückstand mit Ethanol behandelt und das Hydrochlorid abgesaugt, mit Ethanol gewaschen und getrocknet.
   Ausbeute: 105 mg (67 % der Theorie) 7-(S,S-2,8-Diazabicyclo[4.3.0]non-8-yl)-1-(2,4-difluorphenyl)-6,8-difluor-1,4-dihydro-4-oxo-5-vinyl-3-chinolincarbonsäure Hydrochlorid.
   Schmelzpunkt: 224-228°C (unter Zersetzung).
   FAB-Massenspektrum (positiv): m/e 488 [(M+H)⁺], 470 [(488-H₂O)⁺], 975 [(2M+H)⁺].

### Beispiel 13

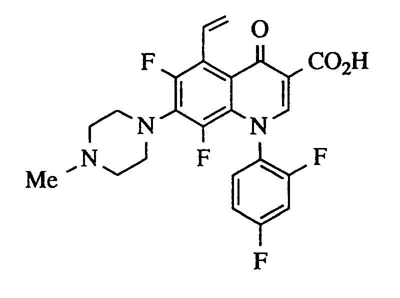

Man setzt analog Beispiel 12 A mit N-Methylpiperazin zu 1-(2,4-Difluorphenyl)6,8-difluor-1,4-dihydro-7-(4-methyl-1-piperazinyl)-4-oxo-5-vinyl-3-chinolincarbonsäure vom Schmelzpunkt 218-220°C (unter Zersetztung) um.

## Patentansprüche

1. Chinolon- und Naphthyridon-carbonsäurederivate der Formel (I) 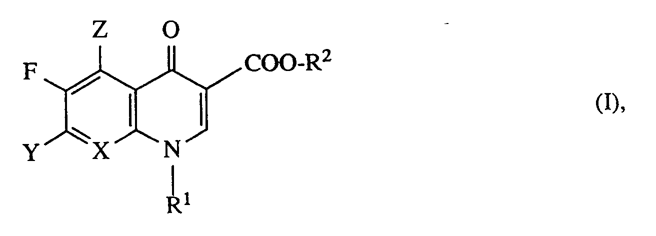 in welcher
R¹ für gegebenenfalls durch Hydroxy, Halogen oder C₁-C₃-Alkoxy substituiertes geradkettiges oder verzweigtes C₁-C₄-Alkyl, für gegebenenfalls durch Halogen oder C₁-C₃-Alkyl substituiertes C₃-C₆-Cycloalkyl, C₂-C₄-Alkenyl, ferner für C₁-C₃-Alkoxy, Amino, Monoalkylamino mit 1 bis 3 C-Atomen, Dialkylamino mit 2 bis 6 C-Atomen oder für gegebenenfalls durch Halogen ein- bis dreifach substituiertes Phenyl steht,
R² für Wasserstoff, Alkyl mit 1 bis 4 Kohlenstoffatomen oder (5-Methyl-2-oxo-1,3-dioxol-4-yl)-methyl steht
X für eine Gruppierung CH, C-Halogen, COCH₃, COCHF₂, C-CH₃ oder N steht,
Z für 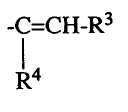 oder -C≡C-R⁵ steht,
worin
R³ Wasserstoff, C₁-C₃-Alkyl, C₁-C₃-Alkoxy oder Alkoxymethyl mit 1 bis 3 C-Atomen im Alkoxyteil,
R⁴ Wasserstoff oder Halogen und
R⁵ Wasserstoff, gegebenenfalls durch Halogen oder Hydroxy ein- bis dreifach substituiertes C₁-C₆-Alkyl, C₂-C₃-Alkenyl, Alkoxy mit 1 bis 3 C-Atomen, Alkoxymethyl mit 1 bis 3 C-Atomen im Alkoxyteil, Halogen oder Trimethylsilyl bedeutet und
Y für 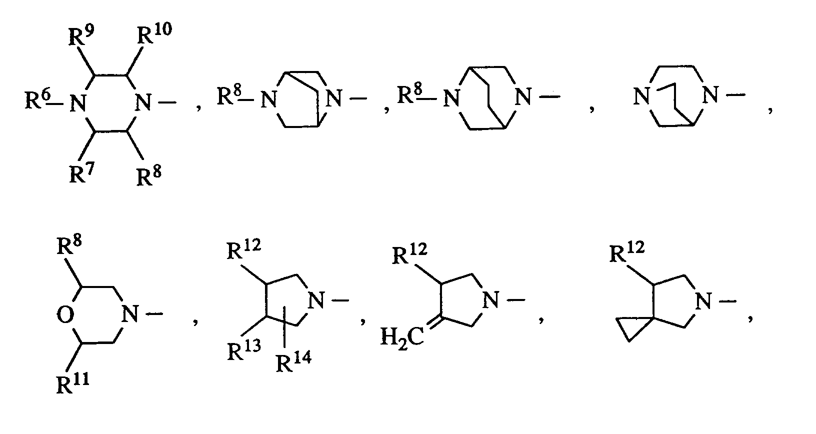 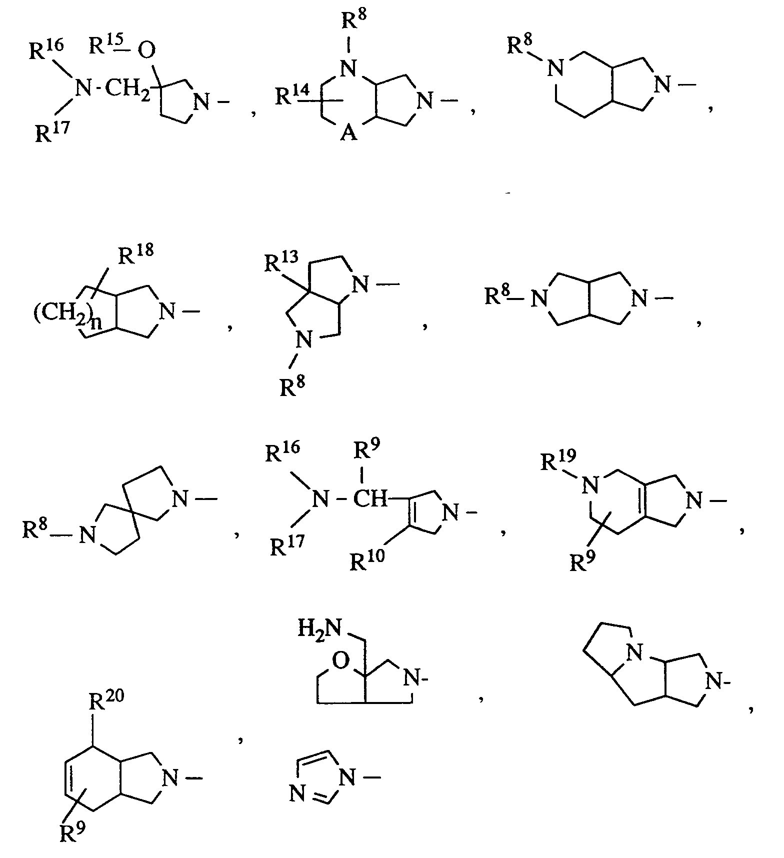 oder 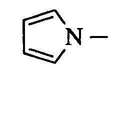 steht,
worin
R⁶ für Wasserstoff, gegebenenfalls durch Hydroxy oder Methoxy substituiertes geradkettiges oder verzweigtes C₁-C₄-Alkyl, Cyclopropyl, Oxoalkyl mit 1 bis 4 C-Atomen, Acyl mit 1 bis 3 C-Atomen,
R⁷ für Wasserstoff, Methyl, Phenyl, Thienyl oder Pyridyl,
R⁸ für Wasserstoff oder Methyl,
R⁹ für Wasserstoff oder Methyl,
R¹⁰ für Wasserstoff oder Methyl,
R¹¹ für Wasserstoff, Methyl oder 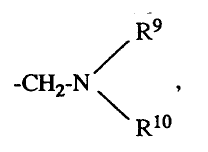
R¹² für Wasserstoff, Methyl, Amino, gegebenenfalls durch Hydroxy substituiertes Alkyl- oder Dialkylamino mit 1 oder 2 C-Atomen im Alkylteil, Aminomethyl, Aminoethyl, gegebenenfalls durch Hydroxy substituiertes Alkyl- oder Dialkylaminomethyl mit 1 oder 2 C-Atomen im Alkylteil oder 1-Imidazolyl,
R¹³ für Wasserstoff, Hydroxy, Methoxy, Methylthio oder Halogen, Methyl, Hydroxymethyl,
R¹⁴ für Wasserstoff oder Methyl,
R¹⁵ für Wasserstoff, Methyl oder Ethyl,
R¹⁶ für Wasserstoff, Methyl oder Ethyl,
R¹⁷ für Wasserstoff, Methyl oder Ethyl,
R¹⁸ für Hydroxy, 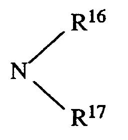 oder 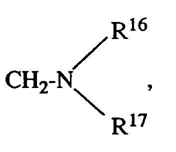
R¹⁹ für Wasserstoff, gegebenenfalls durch Hydroxy substituiertes C₁-C₃-Alkyl, Alkoxycarbonyl mit 1 bis 4 Kohlenstoffatomen im Alkoxyteil oder C₁-C₃-Acyl,
R²⁰ für Wasserstoff, Hydroxy, 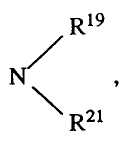 Hydroxymethyl oder 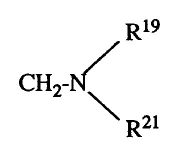 steht, worin
R²¹ Wasserstoff oder Methyl bedeutet,
A für CH₂, O oder für eine direkte Bindung steht und
n für 1 oder 2 steht,
und deren pharmazeutisch verwendbaren Hydrate und Säureadditionssalze sowie die Alkali-, Erdalkali-, Silber- und Guanidiniumsalze.

2. Chinolon- und Naphthyridon-carbonsäurederivate gemäß Anspruch 1,
worin
R¹ für gegebenenfalls durch Hydroxy oder Halogen substituiertes C₁-C₂-Alkyl, C₃-C₅-Cycloalkyl, Vinyl, Amino, Monoalkylamino mit 1 bis 2 C-Atomen, Dialkylamino mit 2 bis 4 C-Atomen oder für gegebenenfalls durch Halogen ein- oder zweifach substituiertes Phenyl steht,
R² für Wasserstoff, Alkyl mit 1 bis 3 Kohlenstoffatomen oder (5-Methyl-2-oxo-1,3-dioxol-4-yl)-methyl steht
X für eine Gruppierung CH, CF, CCl, COCH₃ oder N steht,
Z für 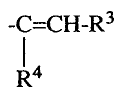 oder -C≡C-R⁵ steht,
worin
R³ Wasserstoff, C₁-C₂-Alkyl, Methoxy oder Methoxymethyl,
R⁴ Wasserstoff und
R⁵ Wasserstoff, gegebenenfalls durch Fluor ein- bis dreifach substituiertes C₁-C₄-Alkyl, C₂-C₃-Alkenyl, Methoxy oder Trimethylsilyl bedeutet und
Y für 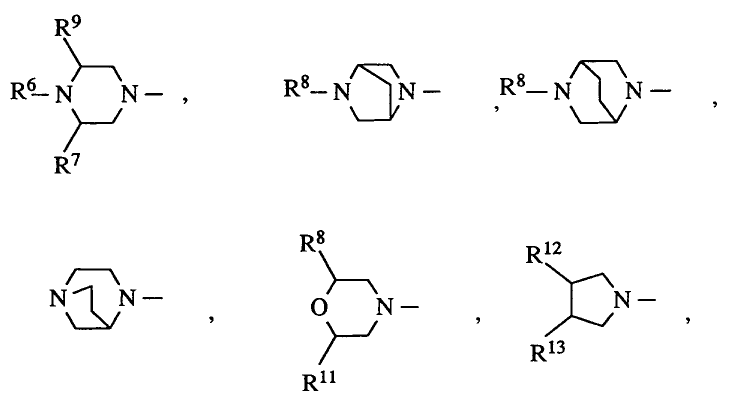 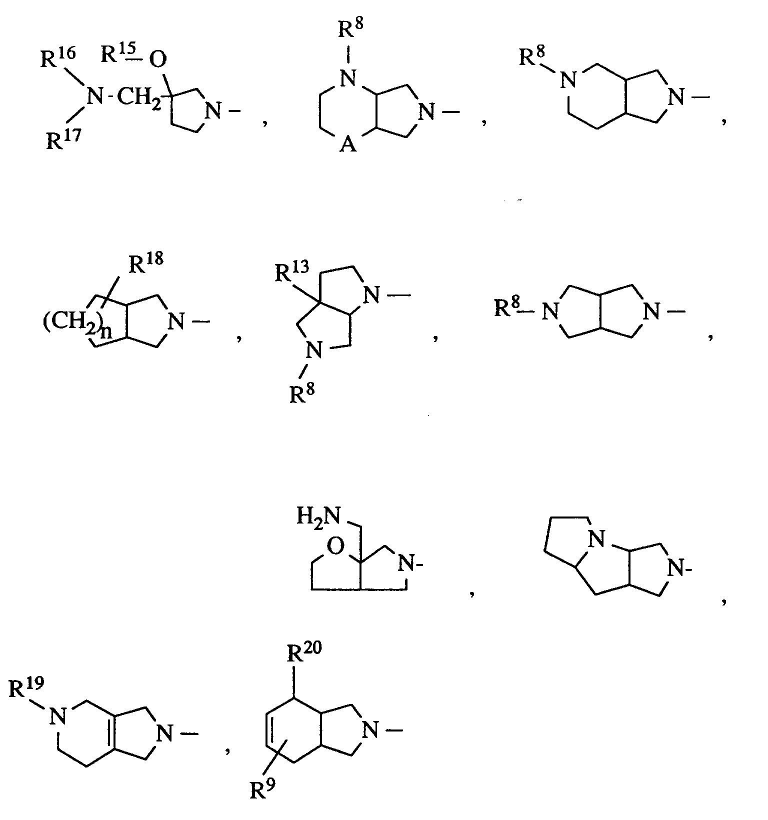 oder 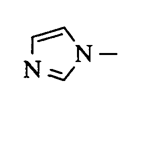 steht,
worin
R⁶ für Wasserstoff, gegebenenfalls durch Hydroxy substituiertes geradkettiges oder verzweigtes C₁-C₃-Alkyl, Oxoalkyl mit 1 bis 4 C-Atomen,
R⁷ für Wasserstoff, Methyl oder Phenyl,
R⁸ für Wasserstoff oder Methyl,
R⁹ für Wasserstoff oder Methyl,
R¹¹ für Wasserstoff, Methyl oder -CH₂-NH₂,
R¹² für Wasserstoff, Methyl, Amino, Methylamino, Dimethylamino, Aminomethyl, Methylaminomethyl oder Ethylaminomethyl,
R¹³ für Wasserstoff, Hydroxy, Methoxy, Fluor, Methyl oder Hydroxymethyl,
R¹⁵ für Wasserstoff oder Methyl,
R¹⁶ für Wasserstoff oder Methyl,
R¹⁷ für Wasserstoff oder Methyl,
R¹⁸ für 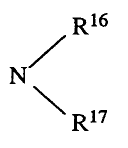 oder 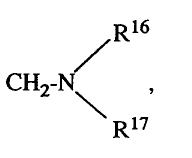
R¹⁹ für Wasserstoff, Methyl oder Ethyl,
R²⁰ für 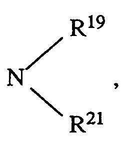 steht,
worin
R²¹ Wasserstoff oder Methyl bedeutet,
A für CH₂, O oder für eine direkte Bindung steht und
n für 1 oder 2 steht.

3. Chinolon- und Naphthyridon-carbonsäurederivate gemäß Anspruch 1,
worin
R¹ für Methyl, Ethyl, Cyclopropyl, Fluorcyclopropyl, oder gegebenenfalls durch Fluor ein- oder zweifach substituiertes Phenyl,
R² für Wasserstoff, Methyl oder Ethyl,
X für eine Gruppierung CH, CF oder CCl steht,
Z für -CH=CH₂ oder C≡C-R⁵ steht,
worin
R⁵ Wasserstoff oder Trimethylsilyl bedeutet und
Y für 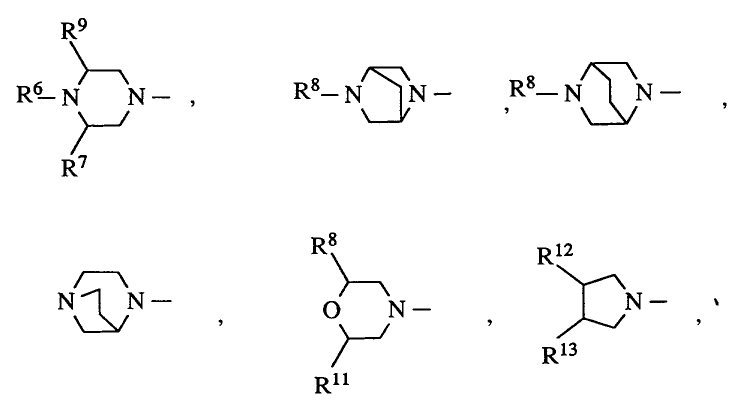 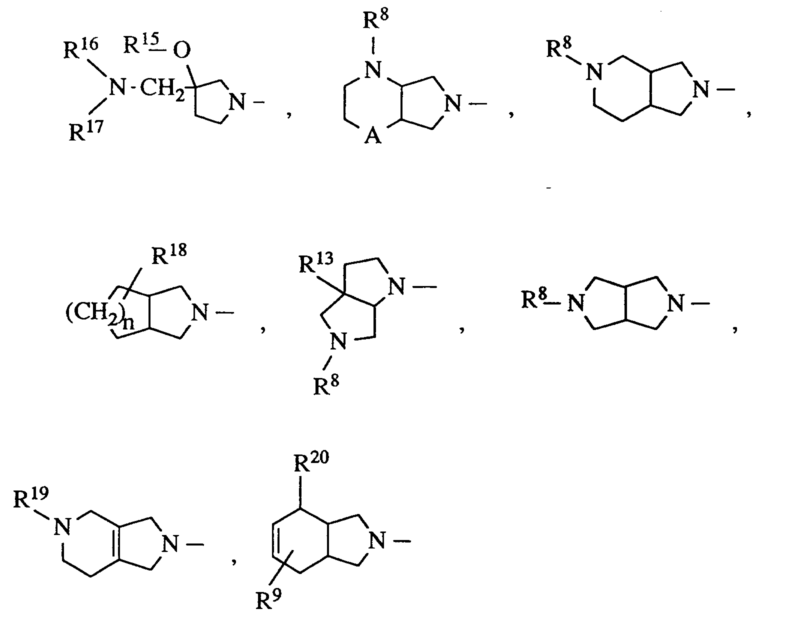 oder 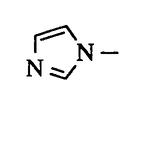 steht,
worin
R⁶ für Wasserstoff, Methyl, gegebenenfalls durch Hydroxy substituiertes Ethyl,
R⁷ für Wasserstoff oder Methyl,
R⁸ für Wasserstoff oder Methyl,
R⁹ für Wasserstoff oder Methyl,
R¹¹ für Wasserstoff oder -CH₂-NH₂,
R¹² für Wasserstoff, Methyl, Amino, Methylamino, Aminomethyl oder Ethylaminomethyl,
R¹³ für Wasserstoff, Hydroxy oder Methoxy,
R¹⁵ für Wasserstoff oder Methyl,
R¹⁶ für Wasserstoff oder Methyl,
R¹⁷ für Wasserstoff oder Methyl,
R¹⁸ für 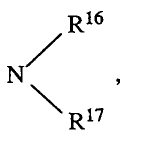
R¹⁹ für Wasserstoff oder Methyl,
R²⁰ für 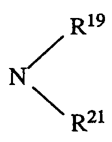 steht,
worin
R²¹ Wasserstoff oder Methyl bedeutet,
A für CH₂, O oder für eine direkte Bindung steht und
n für 1 steht.

4. Chinolon- und Naphthyridon-carbonsäurederivate der Formel (II) 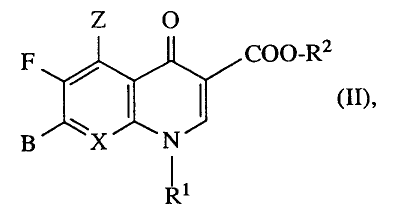 in welcher
R¹, R², X und Z die Bedeutung gemäß Anspruch 1 haben und
B für Halogen, insbesondere Fluor oder Chlor, steht.

5. Verfahren zur Herstellung von Chinolon- und Naphthyridon-carbonsäurederivaten gemäß Anspruch 1, dadurch gekennzeichnet, daß man eine Verbindung der Formel (II) 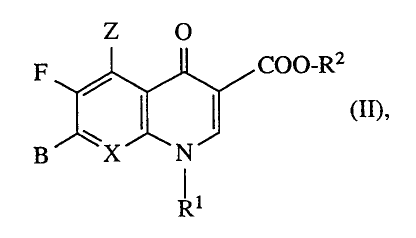 in welcher
R¹, R², X und Z die Bedeutung gemäß Anspruch 1 haben und
B für Halogen, insbesondere Fluor oder Chlor, steht,
mit Verbindungen der Formel (III)
Y-H (III),
in welcher
Y die Bedeutung gemaß Anspruch 1 hat,
gegebenenfalls in Gegenwart von Säurefängern umsetzt.

6. Verfahren zur Herstellung von Chinolon- und Naphthyridon-carbonsäurederivaten gemäß Anspruch 1 , dadurch gekennzeichnet, daß man eine Verbindung der Formel (IV) 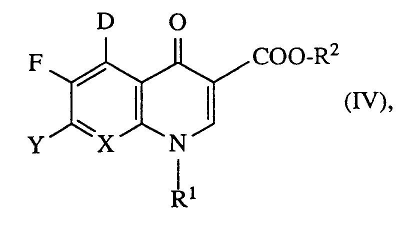 in welcher
R¹, R², X und Y die oben angegebene Bedeutung gemäß Anspruch 1 haben und
D für Halogen, insbesondere Chlor, Brom oder Iod oder eine Grupe OSO₂R_{F} steht, wobei R_{F} für einen Perfluoralkylrest mit 1 bis 8 C-Atomen steht,
mit metallorganischen Vinyl- oder Alkinylverbindungen der Formel (V)
M-Z (V),
in welcher
Z die Bedeutung gemaß Anspruch 1 hat und
M für SnR'₃, ZnX', B(OR'')₂,
worin
R' C₁-C₄-Alkyl,
R'' Wasserstoff oder C₁-C₄-Alkyl und
X' Brom oder Chlor bedeutet,
steht,
in Gegenwart von Übergangsmetall-Katalysatoren umsetzt und gegebenenfalls vorhandene Schutzgruppen abspaltet.

7. Verfahren zur Herstellung von Chinolon- und Naphthridon-carbonsäurederivaten gemäß Anspruch 4, dadurch gekennzeichnet, daß man eine Verbindung der Formel (VI) 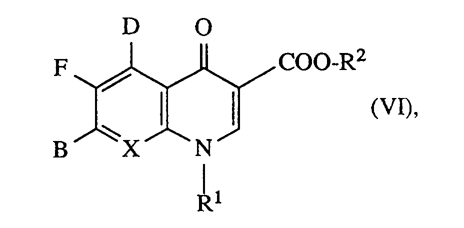 in welcher
R¹, R², X, B und D die Bedeutung gemäß Anspruch 1 bis 5 haben,
mit metallorganischen Vinyl- oder Alkinylverbindungen der Formel (V)
M-Z (V),
in welcher
M und Z die Bedeutung gemäß Anspruch 6 haben,
in Gegenwart von Übergangsmetall-Katalysatoren umsetzt und gegebenenfalls vorhandene Schutzgruppen abspaltet.

8. 1-Cyclopropyl-6,7,8-trifluor-1,4-dihydro-4-oxo-5-vinyl-3-chinolincarbonsäure und deren Ester.

9. 6,7,8-Trifluor-1-(2,4-difluorphenyl)-1,4-dihydro-4-oxo-5-vinyl-3-chinolincarbonsäure und deren Ester.

10. 1-Cyclopropyl-6,7,8-trifluor-1,4-dihydro-5-trimethylsilylethinyl-4-oxo-3-chinolincarbonsäure und deren Ester.

11. 1-Cyclopropyl-5-ethinyl-6,7,8-trifluor-1,4-dihydro-4-oxo-3-chinolincarbonsäure und deren Ester.

12. 1-Cyclopropyl-7-(S,S-2,8-diazabicyclo[4.3.0]non-8-yl)-6,8-difluor-1,4-dihydro-4-oxo-5-vinyl-3-chinolincarbonsäure.

13. 7-(3-Amino-pyrrolidin-1-yl)-1-cyclopropyl-6,8-difluor-1,4-dihydro-4-oxo-5-vinyl-3-chinolincarbonsäure.

14. Chinolon- und Naphthyridon-carbonsäurederivate gemaß Ansprüchen 1-3 zur Bekämpfung von Krankheiten im Human- und Veterinärbereich.

15. Arzneimittel enthaltend Chinolon- und Naphthyridon-carbonsäurederivate gemäß Ansprüchen 1-3.

16. Verwendung von Chinolon- und Naphthyridon-carbonsäurederivaten gemäß Ansprüchen 1-3 bei der Herstellung von Arzneimitteln.
